# EUROPEAN PATENT APPLICATION

(11) **EP 4 342 497 A1**
(43) Date of publication of application: **27.03.2024**
(21) Application number: 22807422.5
(22) Date of filing: 09.05.2022
(51) Int. Cl.: A61K 47/56, A61K 39/395, A61P 25/28

(54) **ANTIBODY HAVING REDUCED BINDING AFFINITY FOR ANTIGEN**

(30) Priority: 10.05.2021 JP 2021079655
(71) Applicant: Kawasaki Institute of Industrial Promotion, Kawasaki-shi, Kanagawa 212-0013 (JP)
(72) Inventor: KATAOKA, Kazunori, Kawasaki-shi, Kanagawa 212-0013 (JP); YANG, Tao, Kawasaki-shi, Kanagawa 212-0013 (JP); MOCHIDA, Yuki, Kawasaki-shi, Kanagawa 212-0013 (JP); ANRAKU, Yasutaka, Kawasaki-shi, Kanagawa 212-0013 (JP); CABRAL, Horacio, Kawasaki-shi, Kanagawa 212-0013 (JP); KINOH, Hiroaki, Kawasaki-shi, Kanagawa 212-0013 (JP)
(74) Representative: Hoffmann Eitle
(86) International application number: PCT/JP2022/019622
(87) International publication number: WO 2022/239720

(57) **Abstract**

The present invention relates to a technique for delivering an antibody to the brain or a tumor tissue. The present invention provides a modified antibody that is targeted. The present invention provides an inactivated antibody that is targeted, wherein the antibody is a modified antibody that is reactivated in the brain or a tumor tissue.

## Description

### Technical Field

The present invention relates to an antibody having reduced binding affinity for an antigen.

### Background Art

Antibody drugs for use in therapy of diseases have been developed worldwide. An antibody is a molecular target drug, and therefore is ideally specific for a target tissue. Actually, however, it is often the case that an antibody is not perfectly specific for a target tissue because an antigen relevant to the antibody is also expressed in areas other than the target tissue. In particular, an anticancer agent comprising an antibody targeting a cancer antigen may cause side effects by damaging areas other than a tumor if the target tissue specificity is low.

Glioblastoma (GBM) which is the most progressive brain tumor is characterized by the highest fatality rate, shortened survival and poor prognosis, and generally causes death (Non Patent Literatures 1 to 3). Immune checkpoint blockade (ICB) therapy using a monoclonal antibody has revolutionized cancer therapy (Non Patent Literatures 4 to 6). However, there is a limit on delivery of an antibody beyond the blood-brain barrier (BBB), and glioblastoma patients are rarely responsive to ICB therapy (Non Patent Literatures 7 to 9). In addition, ICB therapy may cause immune-related adverse events (irAEs) such as activation of autoimmunity, lymphocytic infiltration, and release of inflammatory cytokines (Non Patent Literatures 10 to 13). In ICB therapy, PD-L1 is expressed in vascular endothelial cells, muscle, liver cells and pancreatic islet cells, and off-target binding to a normal tissue constantly occurs. Therefore, if an anti-PD-L1 antibody (aPD-L1) is systemically administered, the incidence of irAEs increases, leading to a decrease in therapeutic effect (Non Patent Literatures 14 to 16).

### Citation List

### Patent Literature

Patent Literature 1: WO 2012/112689 A

### Non Patent Literature

Non Patent Literature 1: Lim, M., Xia, Y., Bettegowda, C. & Weller, M. Current state of immunotherapy for glioblastoma. Nat. Rev. Clin. Oncol. 15, 422-442 (2018).
Non Patent Literature 2: Aldape, K., et al. Challenges to curing primary brain tumours. Nat. Rev. Clin. Oncol. 16, 509-520 (2019).
Non Patent Literature 3: Arvanitis, C.D., Ferraro, G.B. & Jain, R.K. The blood-brain barrier and blood-tumour barrier in brain tumours and metastases. Nat. Rev. Cancer. 20, 26-41 (2020) .
Non Patent Literature 4: Binnewies, M., et al. Understanding the tumour immune microenvironment (TIME) for effective therapy. Nat. Med. 24, 541-550 (2018).
Non Patent Literature 5: Mellman, I., Coukos, G. & Dranoff, G. Cancer immunotherapy comes of age. Nature 480, 480-489 (2011).
Non Patent Literature 6: Pardoll, D.M. The blockade of immune checkpoints in cancer immunotherapy. Nat. Rev. Cancer. 12, 252-264 (2012).
Non Patent Literature 7: Herbst, R.S., et al. Predictive correlates of response to the anti-PD-L1 antibody MPDL3280A in cancer patients. Nature 515, 563-567 (2014).
Non Patent Literature 8: Gajewski, T.F., Schreiber, H. & Fu, Y.-X. Innate and adaptive immune cells in the tumour microenvironment. Nat. Immunol. 14, 1014-1022 (2013).
Non Patent Literature 9: McGranahan, T., Therkelsen, K.E., Ahmad, S. & Nagpal, S. Current state of immunotherapy for treatment of glioblastoma. Curr. Treat. Options in Oncol. 20, 24 (2019).
Non Patent Literature 10: Sury, K., Perazella, M.A. & Shirali, A.C. Cardiorenal complications of immune checkpoint inhibitors. Nat. Rev. Nephrol. 14, 571-588 (2018).
Non Patent Literature 11: Ramos-Casals, M., et al. Immune-related adverse events of checkpoint inhibitors. Nat. Rev. Dis. Primers 6, 38 (2020).
Non Patent Literature 12: Postow, M.A., Sidlow, R. & Hellmann, M.D. Immune-related adverse events associated with immune checkpoint blockade. N. Engl. J. Med. 378, 158-168 (2018).
Non Patent Literature 13: Martins, F., et al. Adverse effects of immune-checkpoint inhibitors: epidemiology, management and surveillance. Nat. Rev. Clin. Oncol. 16, 563-580 (2019).
Non Patent Literature 14: Naidoo, J., et al. Toxicities of the anti-PD-1 and anti-PD-L1 immune checkpoint antibodies. Ann. Oncol. 26, 2375-2391 (2015).
Non Patent Literature 15: Sharpe, A.H., Wherry, E.J., Ahmed, R. & Freeman, G.J. The function of programmed cell death 1 and its ligands in regulating autoimmunity and infection. Nat. Immunol. 8, 239-245 (2007).
Non Patent Literature 16: Rosenberg, J.E., et al. Atezolizumab in patients with locally advanced and metastatic urothelial carcinoma who have progressed following treatment with platinum-based chemotherapy: a single-arm, multicentre, phase 2 trial. The Lancet 387, 1909-1920 (2016).

### Summary of Invention

According to the present invention, there is provided an antibody having reduced binding affinity for an antibody. Further, according to the present invention, there is provided an antibody which is reactivated in an environment-responsive manner.

The present inventors have found that by modifying an antibody with a non-charged hydrophilic polymer to cover the antibody, the affinity of the antibody for an antigen can be reduced to bring the antibody into an inactivated state (a state in which the binding affinity for the antigen is attenuated or lost) in a non-target tissue in the body, and by designing a linker to cleave the modification with the non-charged hydrophilic polymer in a target tissue, so that the modification is removed from the antibody in the target tissue, the antibody can be brought into an activated state (a state in which the affinity for the antigen is further enhanced) only in the target tissue in the body.

The present invention provides the following.
[1] A modified antibody that is modified with a non-charged hydrophilic polymer block (preferably, polyethylene glycol (PEG)), wherein the non-charged hydrophilic polymer block, an environment-responsive bond and the antibody are linked in this order, each of the linkages is optionally via a spacer, and the environment-responsive bond is cleaved under a reducing environment (although not specifically limited to the following, the antibody preferably binds to an immune checkpoint molecule to inhibit an immune checkpoint).
[2] The antibody according to [1], wherein a binding affinity (KD) for an antigen of interest is 100 of that before modification (unmodified antibody).
[3] The antibody according to [1], wherein the binding affinity (KD) for an antigen of interest is 5% of that before modification (unmodified antibody).
[4] The antibody according to [1], which does not substantially or significantly bind to its antigen {for example, the binding is equal to or below a detection limit} under a serum environment.
[5] The antibody according to any one of [1] to [4], wherein the environment-responsive bond is cleaved under a reducing environment in a brain parenchyma or under a reducing environment in a tumor tissue.
[6] The antibody according to [5], wherein when the environment-responsive bond is cleaved, the binding affinity (KD) for its antigen is recovered.
[7] The antibody according to any one of [1] to [6], wherein the antibody has an amino group modified with - C(O)-O-L₁-S-S-L₂-(a non-charged hydrophilic polymer block),
   wherein
   L₁ is an optionally substituted lower alkylene, and
   L₂ is a bond, or a linker stable in a living organism.
[8] The antibody according to [7], wherein L₁ is ethylene.
[9] The antibody according to any one of [1] to [8], wherein the antibody inhibits a PD-1-axis immune checkpoint.
[10] The antibody according to [9], wherein the antibody binds to PD-L1 or PD-1.
[11] The antibody according to any one of [1] to [10], wherein the antibody exposes a targeting molecule.
[12] The antibody according to [11], wherein the targeting molecule is linked to the non-charged hydrophilic polymer block.
[13] The antibody according to [12], wherein the targeting molecule is a GLUT1 ligand.
[14] The antibody according to [13], wherein the GLUT1 ligand is glucose.
[15] The antibody according to any one of [1] to [14], which binds to an immune checkpoint molecule to neutralize interaction between the immune checkpoint molecules.
[16] The antibody according to [15], wherein the immune checkpoint molecule is a counterpart of an immune checkpoint molecule expressed in immune cells.
[17] The antibody according to [15], wherein the immune checkpoint molecule is expressed in immune cells.
[18] A pharmaceutical composition comprising the antibody according to any one of [1] to [17].
[19] The antibody according to any one of [1] to [6], wherein an amino group of an amino acid residue of the antibody is linked to the non-charged hydrophilic polymer block and the environment-responsive bond.
[20] A pharmaceutical composition comprising the antibody according to [18].
[21] The antibody according to any one of [1] to [17] and [19], wherein the antigen is a tumor antigen.
[22] The antibody according to [21], wherein the tumor antigen is a non-brain tumor antigen.
[23] The antibody according to [22], wherein the tumor antigen is a brain tumor antigen.
[24] The antibody according to any one of [1] to [17] and [19] to [23], which exposes a targeting molecule against a target tumor.
[25] The antibody according to [24], wherein the target antigen is linked to the non-charged hydrophilic polymer block.
[26] A pharmaceutical composition comprising the antibody according to any one of [1] to [17] and [19] to [25].
[27] The pharmaceutical composition according to [26], for use in treating cancer.
[28] An antigen-binding fragment of the antibody according to any one of the above.
[29] A pharmaceutical composition comprising the antigen-binding fragment according to [28].
[30] The antibody according to any one of the above, wherein the antigen is in a form of an antibody-drug conjugate (ADC).
[31] The antibody according to [30], wherein an average hydrodynamic diameter (in volume average) is 6 nm to 12 nm (12 nm or less than 12 nm).
[32] The antibody according to [31], wherein an average hydrodynamic diameter (in volume average) of unmodified ADC is 12 nm or less.
[33] The antibody according to any one of [30] to [32], wherein ADC is obtained by introducing a -linker-drug moiety to a side-chain amino group of an antibody moiety.
[34] The antibody according to any one of [30] to [33], wherein ADC is obtained by introducing two to four - linker-drug moieties to the side-chain amino group of the antibody moiety, that is, ADC is represented by antibody moiety-(linker-drug)n {where n is 2 to 10 as a number average}.
[35] The antibody according to [30], wherein ADC is obtained by introducing two to four -linker-drug moieties to the side-chain amino group of the antibody moiety, and has an average hydrodynamic diameter (in volume average) of 12 nm to 30 nm (in particular, 13 nm to 25 nm).
[36] The antibody according to [35], wherein the average hydrodynamic diameter (in volume average) of unmodified ADC is 12 nm or less.
[37] The antibody according to any one of the above, wherein the antigen to which the antibody moiety binds is a cancer antigen.
[38] A composition comprising the antibody according to any one of the above.
[39] A composition for use in intracorporal targeting of an antigen to which the antibody moiety binds, the composition comprising the antibody according to any one of above.
[40] The antibody according to any one of above, wherein ADC comprises one or more selected from the group consisting of gemtuzumab ozogamicin (Mylotarg), ibritumomab tiuxetan (Zevalin), brentuximab vedotin (Adcetris), trastuzumab emtansine (KADCYLA), inotuzumab ozogamicin (BESPONSA), moxetumomab pasudotox-tdfk (LUMOXITI), polatuzumab vedotin-Piiq (Polivy), trastuzumab deruxtecan (ENHERTU) and enfortumab vedotin (PADCEV) .
[41] A composition comprising the antibody according to [40] .
[42] The composition according to [41], for use in treatment of cancer.

[1] An antibody (for example, a modified antibody) that binds to an immune checkpoint molecule to inhibit an immune checkpoint, wherein the antibody is modified with a non-charged hydrophilic polymer block (preferably, polyethylene glycol (PEG)), the non-charged hydrophilic polymer block, an environment-responsive bond and the antibody are linked in this order, each of the linkages is optionally via a spacer, and the environment-responsive bond is cleaved under a reducing environment.
[2] The antibody according to [1], wherein a binding affinity (KD) for an antigen of interest is 10% or less of that before modification.
[3] The antibody according to [1], wherein the binding affinity (KD) for an antigen of interest is 5% or less of that before modification.
[4] The antibody according to [1], which does not substantially or significantly bind to its antigen {for example, the binding is equal to or below a detection limit} under a serum environment.
[5] The antibody according to any one of [1] to [4], wherein the environment-responsive bond is cleaved under a reducing environment in a brain parenchyma or under a reducing environment in a tumor tissue.
[6] The antibody according to [5], wherein when the environment-responsive bond is cleaved, the binding affinity (KD) for an antigen of interest is recovered.
[7] The antibody according to any one of [1] to [6], wherein the antibody has an amino group modified with - C(O)-O-L₁-S-S-L₂- (a non-charged hydrophilic polymer block),
   wherein
   L₁ is an optionally substituted lower alkylene, and
   L₂ is a bond, or a linker stable in a living organism.
[8] The antibody according to [7], wherein L₁ is ethylene.
[9] The antibody according to any one of [1] to [8], which inhibits a PD-1-axis immune checkpoint.
[10] The antibody according to [9], which binds to PD-L1 or PD-1.
[11] The antibody according to any one of [1] to [10], which exposes a targeting molecule.
[12] The antibody according to [11], wherein the targeting molecule is linked to the non-charged hydrophilic polymer block.
[13] The antibody according to [12], wherein the targeting molecule is a GLUT1 ligand.
[14] The antibody according to [13], wherein the GLUT1 ligand is glucose.
[15] The antibody according to any one of [1] to [14], the antibody that binds to an immune checkpoint molecule to inhibit an immune checkpoint is an antibody that binds to an immune checkpoint molecule to neutralize interaction between the immune checkpoint molecules.
[16] The antibody according to [15], wherein the immune checkpoint molecule is a counterpart of an immune checkpoint molecule expressed in immune cells.
[17] The antibody according to [15], wherein the immune checkpoint molecule is expressed in immune cells.
[18] A pharmaceutical composition comprising the antibody according to any one of [1] to [17].
[19] The antibody according to any one of [1] to [6], wherein an amino group of an amino acid residue of the antibody is linked to the non-charged hydrophilic polymer block and the environment-responsive bond.
[20] A pharmaceutical composition comprising the antibody according to [18].
[21] The pharmaceutical composition according to [20], for use in treating cancer.

### Brief Description of Drawings

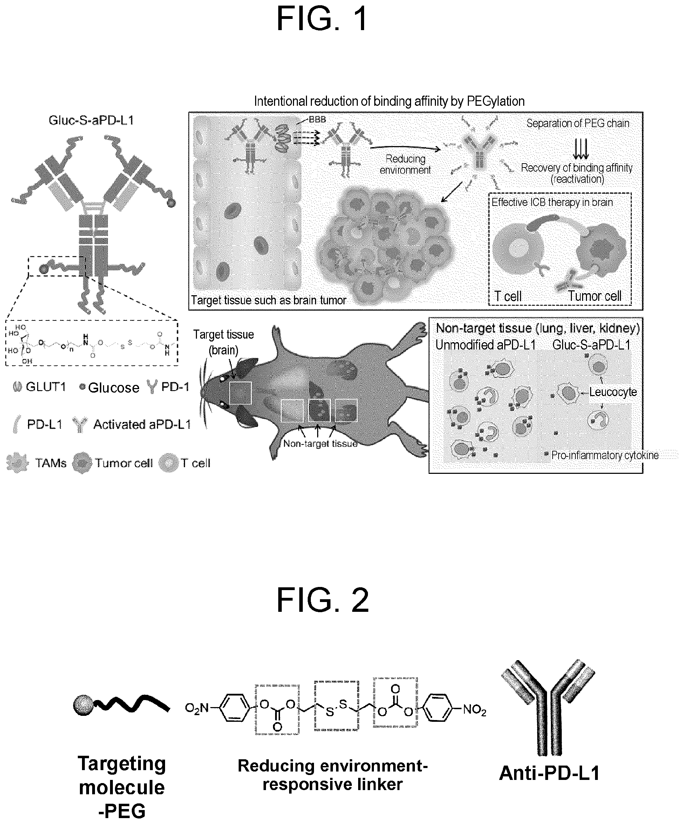

[Figure 1] Figure 1 shows an embodiment of an antibody of the present invention, and a behavior of the antibody in a living organism. Specifically, in the antibody in the embodiment, an antibody and a non-charged hydrophilic polymer block are linked via a reducing environment-responsive linker. The binding affinity of the antibody for an antigen is reduced or eliminated by linkage to the non-charged hydrophilic polymer block. Thus, the antibody is inactivated in areas other than a target organ (including non-target tissues). In Figure 1, an example is shown in which the antibody is delivered to a brain, and functions in the brain. As shown in Figure 1, for example, when the antibody moves to the brain from the blood, the reducing environment-responsive linker is cleaved in response to a reducing environment in a brain parenchyma. The cleavage causes separation of the non-charged hydrophilic polymer block from the antibody, upon which the antibody is released from steric hindrance by the non-charged hydrophilic polymer block, and recovers its binding affinity for the original antigen of interest. In this way, in the present invention, the antibody can be reactivated to exert an action against an antigen of interest only in the brain and a tumor tissue, and other special environments where a sulfide bond is cleaved.
[Figure 2] Figure 2 shows a specific schematic diagram according to an embodiment of an antibody of the present invention. An anti-PD-L1 antibody and a non-charged hydrophilic polymer block having a targeting molecule at one end thereof (for example, PEG) are linked via a reducing environment-responsive linker.
[Figure 3] Figure 3 shows the results of determining the amount of unmodified side-chain amino groups in a modified antibody. From the fact that the amount of unmodified side-chain amino groups decreases after modification, it is indicated that a non-charged hydrophilic polymer block binds to the antibody via the amino groups of the antibody.
[Figure 4] Figure 4 demonstrates that the size of a modified antibody of the present invention with reduced binding affinity decreases in size with a modification removed from the antibody under a reducing environment, and substantially returns to an unmodified antibody size (left panel). The right panel demonstrates that the antibody is modified with a non-charged hydrophilic polymer block to lose a zeta potential, and released from the non-charged hydrophilic polymer block in a reducing environment to recover the zeta potential.
[Figure 5] Figure 5 shows a structure of a linker in a preferred embodiment of the present invention, and a naive antibody (unmodified antibody) generated or released by cleavage of the linker in a reducing environment when the linker is used.
[Figure 6] Figure 6 shows an embodiment of an antibody of the present invention in which the binding affinity for an antigen of interest is recovered in a reducing environment-dependent manner. Gluc-S-aPD-L1 substantially loses binding affinity for an antigen (see the middle bar), whereas the binding affinity is recovered to an unmodified antibody level under a reducing environment (Gluc-S-aPD-L1/GSH).
[Figure 7] Figure 7 shows stained cell images demonstrating that an antibody recovering the binding ability under a reducing environment recognizes PD-L1 on cell surfaces (upper panel), and a graph showing the amount of binding (fluorescence intensity).
[Figure 8] Figure 8 shows the retainability of an antibody in the blood (lower left panel) and the amount of the antibody in the plasma (lower right panel). An unmodified anti-PD-L1 antibody exhibits binding to the inner wall of a blood vessel, whereas a modified antibody does not exhibit substantial binding to the inner wall of a blood vessel.
[Figure 9] An antibody modified with a non-charged hydrophilic polymer block, wherein 0%, 25%, 50% or 100% of the non-charged hydrophilic polymer blocks have glucose at one end thereof, was obtained. Figure 9 shows the accumulations of various antibodies in a brain tumor.
[Figure 10-1] Figure 10-1 shows the antitumor effects of various antibodies on proliferation of a tumor (GL261 cell line) in mice with the tumor transplanted into one side of the brain.
[Figure 10-2] Figure 10-2 shows the antitumor effects of various antibodies on proliferation of a tumor (GL261 cell line) in mice with the tumor transplanted into one side of the brain. The left panel shows a Kaplan-Meier curve showing a viability, and the right panel shows a change in body weight of treated mice.
[Figure 11] Figure 11 shows the antitumor effects of various antibodies on proliferation of a tumor (CT-2A cell line) in mice with the tumor transplanted into one side of the brain.
[Figure 12] Figure 12 shows a schematic diagram of a system in which an antibody according to an embodiment of the present invention is detected with a secondary antibody. In Figure 12, the antibody is prevented from being accessed by the secondary antibody by modification with a non-charged hydrophilic polymer block, so that recognition of the antibody by the secondary antibody is inhibited. On the other hand, the antibody released from the non-charged hydrophilic polymer block is recognized by the secondary antibody.
[Figure 13] Figure 13 demonstrates that in a brain tumor, a modification is removed from an antibody according to an embodiment of the present invention, so that the antibody of the present invention is recognized by a secondary antibody.
[Figure 14-1] Figure 14-1 shows the results of flow cytometry which show that the number of cytotoxic T-cells increases in the brains of mice treated with various antibodies.
[Figure 14-2] Figure 14-2 shows the results of immunohistochemical staining which show that the number of cytotoxic T-cells increases in the brain of mice treated with various antibodies.
[Figure 14-3] Figure 14-3 shows the blood concentrations of interferon γ (IFNγ) after stimulation of antigen processing and presentation mechanisms in mice receiving various antibodies.
[Figure 15] Figure 15 shows the abundance of immunosuppressive Foxp3 + T-cells in mice receiving various antibodies.
[Figure 16] Figure 16 shows the abundance of CD44hiCD62L low effector memory T-cell subsets in the spleen of mice receiving various antibodies.
[Figure 17-1] Figure 17-1 shows a scheme in which an antibody according to an embodiment of the present invention is administered to a mouse with a tumor grafted into the right brain, a tumor is then grafted into the left brain, and the tumors are observed.
[Figure 17-2] Figure 17-2 shows the presence of the tumor in the left brain (left panel), and a Kaplan-Meier curve (right panel).
[Figure 18] Figure 18 shows infiltration of CD45+cells in non-target tissues (lung, kidney and liver) of mice receiving various antibodies.
[Figure 19] Figure 19 shows TNF-α, IL-6 and IL-1β levels in non-target tissues (lung, kidney and liver) of mice receiving various antibodies.
[Figure 20] Figure 20 demonstrates that in non-target tissues (lung, kidney and liver) of mice receiving various antibodies, an antibody according to an embodiment of the present invention cannot be recognized by a secondary antibody, that is, the antibody is inactive without removal of a modification from the antibody in the non-target tissues.
[Figure 21-1] Figure 21-1 shows a schematic diagram of a modified antibody which have no targeting molecule and in which a non-charged hydrophilic polymer block (for example, PEG), a reducing environment-responsive linker and an antibody are linked in this order.
[Figure 21-2] Figure 21-2 shows an antitumor effect when having no targeting molecule and having a configuration shown in Figure 21-1 is administered to a cancer subcutaneous transplantation model.
[Figure 22] Figure 22 shows a distribution of the hydrodynamic diameters of unmodified T-DM1 and PEG-modified T-DM1.
[Figure 23] Figure 23 shows a time-dependent change in particle diameter distribution of the PEG-modified antibody and a change in scattering light intensity under a reducing environment simulating a reducing environment in a cancer tissue in a living organism.

### Description of Embodiments

In the present specification, the term "subject" refers to a mammal including a human. The subject may be a healthy subject, or a subject affected by some disease.

In the present specification, the term "treatment" is used in the sense of including both therapeutic treatment and prophylactic treatment. In the present specification, the term "therapy" means therapy of a disease or disorder, cure, prevention or improvement of remission, or reduction of the progression rate of a disease or disorder. In the present specification, the term "prophylaxis" means that the possibility of the onset of a disease or pathological condition is reduced, or the onset of a disease or pathological condition is delayed.

In the present specification, the term "disease" means a symptom for which therapy is beneficial. In the present specification, the cancer generally means a tumor with a high degree of malignancy, such as a malignant tumor or a brain tumor. The tumor includes a benign tumor or a malignant tumor.

In the present specification, the term "blood-brain barrier" refers to a functional barrier which is present between the blood (or circulated blood) and the brain, and has selectivity on permeation of a substance. The blood-brain barrier is considered to be formed by cerebrovascular endothelial cells. Most of the substance permeability of the blood-brain barrier is unclear, but glucose, alcohol and oxygen are known to easily pass through the blood-brain barrier, and it is considered that lipid-soluble substances and small molecules (for example, molecules having a molecular weight of less than 500) tend to more easily pass through the blood-brain barrier as compared to water-soluble molecules and high-molecular-weight molecules (for example, molecules having a molecular weight of 500 or more). Many therapeutic drugs for brain diseases and cerebral diagnostics do not pass through the blood-brain barrier, and this is a major obstacle to therapy of brain diseases, brain examination and the like. In the present specification, the term "blood-nerve barrier" refers to a functional barrier which is present between the circulated blood and the peripheral nerve, and has selectivity on permeation of a substance. In the present specification, the term "blood-spinal fluid barrier" refers to a functional barrier which is present between the circulated blood and the cerebral spinal fluid, and has selectivity on permeation of a substance. In the present specification, the term "blood-retina barrier" refers to a functional barrier which is present between the circulated blood and a retinal tissue, and has selectivity on permeation of a substance. It is considered that the blood-nerve barrier, the blood-spinal fluid barrier and the blood-retina barrier are formed by vascular endothelial cells present in the respective barriers, and are similar in function to the blood-brain barrier.

In the present specification, the term "targeting molecule" refers to a molecule binding to a target molecule present on, for example, the surfaces of cells in a living organism (for example, an antigen such as a protein on cell surfaces) in an environment inside the living organism where the target molecule is present. The targeting molecule has binding affinity for the target molecule, and therefore may exhibit an effect of actively delivering (concentrating) a targeting molecule-linked substance to a location where the target molecule is present. The targeting molecule may be a molecule having binding affinity for a target molecule expressed on cell surfaces (for example, endothelial cell surfaces) (for example, an antibody, an antigen-binding fragment thereof, aptamer, peptide, or lectin). An antibody having no targeting molecule or an antibody modified with a non-charged hydrophilic polymer block is passively delivered to a tissue (for example, a tumor tissue) by, for example, the EPR effect. An antibody modified with a non-charged hydrophilic polymer block which has a targeting molecule can bind to a target molecule in an environment in a living organism. The term "targeting molecule" as used herein refers to a molecule moiety which may be linked to an antibody moiety to be modified according to the present invention (preferably to the distal end of the non-charged hydrophilic polymer block; herein, the proximal end is an end linked to the antibody, and the distal end is an end which is not linked to the antibody), and is different from the antibody moiety.

In the present specification, the term "GLUT1 ligand" means a substance binding specifically to GLUT1. As the GLUT1 ligand, various ligands are known, and examples thereof include, but are not limited to, the molecules of glucose, hexose and the like. The GLUT1 ligands can be all used for preparation of an antibody modified with a non-charged hydrophilic polymer block in the present invention. The GLUT1 is preferably comparable or superior in affinity for GLUT 1 to glucose. It is known that 2-N-4-(1-azi-2,2,2-trifluoroethyl)benzoyl-1,3-bis(D-mannose-4-yloxy)-2-propylamine (ATB-BMPA), 6-(N-(7-nitrobenz-2-oxa-1,3-diazol-4-yl)amino)-2-deoxyglucose (6-NBDG), 4,6-O-ethylidene-α-D-glucose, 2-deoxy-D-glucose and 3-O-methylglucose also bind to GLUT1. These molecules can also be used as GLUT1 ligands in the present invention. The GLUT1 ligand includes a GLUT1-binding molecule, and the GLUT1-binding molecule includes a GLUT1-binding aptamer. A GLUT1 ligand having higher specificity for GLUT1 may be more preferably used. In a certain preferred example, the GLUT1 ligand may be glucose.

In the present specification, the term "antibody" means an immunoglobulin, which is a protein having a structure in which two heavy chains (H chains) and two light chains (L chains) stabilized by a pair of disulfide bonds are associated. The heavy chain comprises a heavy chain variable region VH, heavy chain constant regions CH1, CH2 and CH3, and a hinge region located between CH1 and CH2. The light chain comprises a light chain variable region VL and a light chain constant region CL. Among them, VH and VL form a variable region fragment (Fv), which is a region that is directly involved in antigen binding and gives diversity to the antibody. An antigen-binding region comprising VL, CL, VH and CH1 is called a Fab region, and a region comprising the hinge region, CH2 and CH3 is called a Fc region.

Among the variable regions, regions that are in direct contact with an antigen have a particularly large variation, and are each called a complementary-determining region: CDR). Portions other than CDR, which have a relatively small variation, are each called a framework region (FR). The variable regions of the light chain and the heavy chain each have three CDRs, which are called heavy chain CDRs 1 to 3 or light chain CDRs 1 to 3, respectively, in this order from the N-terminal side.

In the present specification, the term "modified antibody" means an antibody having a chemical modification. Examples of the chemical modification include modifications with a non-charged hydrophilic polymer (for example, polyethylene glycol or polyoxazoline).

In the present specification, the antibody may be a monoclonal antibody, or a polyclonal antibody. In the present specification, the antibody may be any of the isotypes of IgG, IgM, IgA, IgD and IgE. The antibody may be prepared by immunizing a non-human animal such as a mouse, a rat, a hamster, a guinea pig, a rabbit or chicken, or may be a recombinant antibody, or a chimeric antibody, a humanized antibody, a fully humanized antibody or the like. The term "chimeric antibody" refers to an antibody obtained by linking fragments of antibodies derived from different species.

The term "humanized antibody" means an antibody in which an amino acid sequence characteristic of an antibody of non-human origin replaces an amino acid sequence at a corresponding location of a human antibody. Examples thereof include antibodies which have heavy chain CDRs 1 to 3 and light chain CDRs 1 to 3 of an antibody prepared by immunizing a mouse or a rat and in which all other regions including four framework regions (FRs) of each of the heavy chain and the light chain are derived from a human antibody. Such an antibody may be called a CDR-grafted antibody. The term "humanized antibody" may include a human chimeric antibody.

The "human chimeric antibody" is an antibody of non-human origin in which the constant regions of the antibody of non-human origin are replaced by the constant regions of a human antibody.

The antibody can be isolated. In a pharmaceutical product, the antibody is preferably an isolated monoclonal antibody. The antibody may have antibody-dependent cellular cytotoxicity (ADCC) and/or complement-dependent cytotoxicity (CDC).

In the present specification, the term "antigen-binding fragment of an antibody" refers to a fragment of an antibody, which maintains an antigen-binding ability. Specifically, examples thereof include, but are not limited to, Fab comprising VL, VH, CL and CH1 regions and Fab' having a hinge region; F(ab')2 in which two Fabs are linked at a hinge region by a disulfide bond; Fv comprising VL and VH; scFv which is a single-chain antibody with VL and VH linked by an artificial polypeptide linker; and multispecific (or bispecific) antibodies of diabody type, scDb type, tandem scFv type and leucine zipper type.

In the present specification, the term "CDR" refers to a complementarity-determining region present in the heavy chain variable region and the light chain variable region of the antibody. The heavy chain and light chain variable regions each have three CDRs, which are called CDR1, CDR2 and CDR3, respectively, in this order from the N-terminal. CDR may be determined on the basis of, for example, the numbering scheme by Kabat et al (Kabat, E.A. et al., Sequences of Proteins of Immunological Interest, 5th ed., 1991, Bethesda: US Dept. of Health and Human Services, PHS, NIH.)

In the present specification, the term "antigen" refers to a substance to which an antibody can bind. The antigen can have immunogenicity. The antigen may be a protein, a nucleic acid, a metabolite or the like.

In the present specification, the term "alkyl" means a linear (i.e., non-branched) or branched carbon chain, or a combination thereof. The alkyl is not limited, and examples thereof include methyl, ethyl, n-propyl, isopropyl, n-butyl, t-butyl, isobutyl, n-pentyl, n-hexyl, n-heptyl, n-octyl, and isomers thereof. In the present specification, the alkyl may be a C₁ alkyl, a C₂ alkyl, a C₃ alkyl, a C₄ alkyl, a C₅ alkyl, a C₆ alkyl, a C₇ alkyl, or a C₈ alkyl. These alkyls may be collectively referred to as a C₁-C₈ alkyl.

In the present specification, the term "alkenyl" refers to a group having a double bond between two adjacent carbon atoms of an alkyl. In the present specification, the term "alkynyl" refers to a group having a triple bond between two adjacent carbon atoms of an alkyl.

In the present specification, the term "lower" means that the number of carbon atoms is 1 to 8, for example, 1 to 6, 1 to 5, 1 to 4, 1 to 3, 1 or 2, 2 to 6, 2 to 5, 2 to 4, or 2 or 3.

In the present specification, the term "optionally substituted" means that a compound or a group may, or not required to, be substituted with another group (i.e., substituted or unsubstituted). Examples of the other group include halogens, a hydroxyl group, lower alkyl groups, and preferably negatively charged groups, for example, a carboxyl group.

In the present specification, the phrase "performing control to obtain a low blood sugar condition" or "lowering the blood sugar level" means that the blood sugar level is made lower than the blood sugar which would be presented if relevant treatment was not performed on a subject. The low blood sugar may be equal to or higher than a level (for example, 70 mg/dL) at which an autonomic nervous symptom such as fatigue, hand tremor, palpitations, fast pulse or cold sweat does not occur. The low blood sugar may be a level at which a central nervous system symptom (for example, a strong sense of exhaustion, fatigue, visual blurriness, headache or drowsiness) or a decline in cerebral function (for example, a decreased level of consciousness, an abnormal behavior, spasm or coma) does not occur. The blood sugar level can be lowered to, for example, about 80 to 100 mg/dL. The term "induction of low blood sugar" is used in the sense of including development of fasting blood sugar. The low blood sugar can be induced by, for example, fasting. Examples of the method for performing control to obtain a low blood sugar condition include administration of a diabetes drug. For example, in performing control to obtain a low blood sugar condition, for example, intake of another drug or drinking of a beverage such as water is allowable as long as the purpose of performing control to obtain a low blood sugar condition is achieved. Induction of low blood sugar may be accompanied by another treatment which has substantially no effect on blood sugar.

In the present specification, the term "fasting" means that a subject is fasted, for example, for 3 hour or more, 4 hour or more, 5 hour or more, 6 hour or more, 7 hour or more, 8 hour or more, 9 hour or more, 10 hour or more, 11 hour or more, 12 hour or more, 13 hour or more, 14 hour or more, 15 hour or more, 16 hour or more, 17 hour or more, 18 hour or more, 19 hour or more, 20 hour or more, 21 hour or more, 22 hour or more, 23 hour or more, 24 hour or more, 25 hour or more, 26 hour or more, 27 hour or more, 28 hour or more, 29 hour or more, 30 hour or more, 31 hour or more, 32 hour or more, 33 hour or more, 34 hour or more, 35 hour or more, 36 hour or more, 37 hour or more, 38 hour or more, 39 hour or more, 40 hour or more, 41 hour or more, 42 hour or more, 43 hour or more, 44 hour or more, 45 hour or more, 46 hour or more, 47 hour or more, or 48 hour or more. The fasting causes the subject to have low blood sugar. The fasting period is determined by a physician or the like in view of the health condition of the subject, and is preferably a period that is longer than a time until the subject reaches fasting blood sugar, for example. The fasting period may be, for example, a time until expression of GLUT1 on the surfaces of cerebrovascular endothelial cells in the blood vessel increases or reaches a plateau. The fasting period may be, for example, 12 hours or more, 24 hours or more or 36 hours or more as described above. The fasting may be accompanied by another treatment which has substantially no effect on the blood sugar level and expression of GLUT1 on the surfaces in the blood vessel.

In the present specification, the phrase "inducing an increase in blood sugar level" means that the blood sugar level is increased in a subject in which control is performed to obtain a low blood sugar condition, or a subject maintaining a low blood sugar condition. The blood sugar level can be increased by various methods well known to those skilled in the art, for example, administration of a substance that induces an increase in blood sugar level, for example, administration of a monosaccharide that induces an increase in blood sugar level, such as glucose, fructose (fruit sugar) or galactose, administration of a polysaccharide that induces an increase in blood sugar level, such as maltose, intake of a carbohydrate that induces an increase in blood sugar level, such as starch, or meals.

In the present specification, the term "blood sugar manipulation" means that in a subject, control is performed to obtain a low blood sugar condition (or a low blood sugar condition is maintained), and the blood sugar level is then increased. After control is performed to obtain a low blood sugar condition in the subject, the blood sugar level of the subject can be kept low. The time during which the blood sugar level of the subject is kept low may be, for example, 0 hour or more, 1 hour or more, 2 hours or more, 3 hour or more, 4 hour or more, 5 hour or more, 6 hour or more, 7 hour or more, 8 hour or more, 9 hour or more, 10 hour or more, 11 hour or more, 12 hour or more, 13 hour or more, 14 hour or more, 15 hour or more, 16 hour or more, 17 hour or more, 18 hour or more, 19 hour or more, 20 hour or more, 21 hour or more, 22 hour or more, 23 hour or more, 24 hour or more, 25 hour or more, 26 hour or more, 27 hour or more, 28 hour or more, 29 hour or more, 30 hour or more, 31 hour or more, 32 hour or more, 33 hour or more, 34 hour or more, 35 hour or more, 36 hour or more, 37 hour or more, 38 hour or more, 39 hour or more, 40 hour or more, 41 hour or more, 42 hour or more, 43 hour or more, 44 hour or more, 45 hour or more, 46 hour or more, 47 hour or more, or 48 hour or more. Thereafter, the blood sugar level can be increased. In the present specification, the "maintenance of blood sugar" means that intake of another drug or drinking of a beverage such as water is allowable as long as the purpose of maintaining low blood sugar in a subject is achieved. Induction of low blood sugar may be accompanied by another treatment which has substantially no effect on the blood sugar. The low blood sugar is not pathological low blood sugar, and it is preferable to have blood sugar sufficient for maintaining consciousness. The low blood sugar may mean, for example, the same level of blood sugar as fasting blood sugar. The level of blood sugar is only required to be low enough to promote the subsequent passage of a modified antibody exposing a GLUT1 ligand through BBB by manipulation for increasing the blood sugar.

Carriers whose outer surfaces are modified with a GLUT1 ligand (for example, glucose) are accumulated in the brain only by administration to a subject (see WO 2015/075942A). Therefore, a dosing regimen according to the present invention does not require fasting or induction of low blood sugar, and/or does not require induction of an increase in blood sugar level. When carriers whose outer surfaces are modified with glucose so as to expose glucose to the surfaces, specifically vesicles such as micelles or polyion-complex polymersomes (PICsome), are administered in accordance with a dosing regimen, these carriers are evidently delivered into the brain (brain parenchyma part) beyond the blood-brain barrier (see WO 2015/075942A). Therefore, it is preferable that the dosing regimen according to the present invention include administering the composition to a subject fasted or induced to have low blood sugar, and it is more preferable that the dosing regimen according to the present invention include administering the composition to a subject fasted or induced to have low blood sugar, and inducing an increase in blood sugar level in the subject. In the dosing regimen according to the present invention, the composition may be administered simultaneously, successively or sequentially to induction of an increase in blood sugar level in the subject. The dosing regimen may, but is not required to, have an interval between administration of the composition to the subject and induction of an increase in blood sugar level in the subject. When the composition is administered simultaneously to induction of an increase in blood sugar level in the subject, the composition may be administered to the subject in a form mixed with a drug causing induction of an increase in blood sugar level, or may be administered in a form separate from a drug causing induction of an increase in blood sugar level in the subject. When the composition is administered successively or sequentially to induction of an increase in blood sugar level in the subject, the composition may be administered to the subject before or after induction of an increase in blood sugar level in the subject. Preferably, the composition can be administered to the subject before induction of an increase in blood sugar level in the subject. When an increase in blood sugar level is induced in the subject before administration of the composition to the subject, it is preferable to administer the composition to the subject within 1 hour, within 45 minutes, within 30 minutes, within 15 minutes or within 10 minutes after induction of an increase in blood sugar level in the subject. When an increase in blood sugar level is induced in the subject after administration of the composition to the subject, it is preferable to induce an increase in blood sugar level within 6 hours, within 4 hours, within 2 hours, within 1 hour, within 45 minutes, within 30 minutes, within 15 minutes or within 10 minutes after administration of the composition to the subject. Two or more cycles of the dosing regimen may be performed. The order relation between administration of glucose and administration of the sample can be determined by the timing of passage through the blood-brain barrier. In the present invention, administration of glucose can be replaced by meal intake. According to WO 2015/075942A, it has been revealed that micelles pass through BBB to accumulate in the brain even without blood sugar manipulation. In a certain embodiment of the present invention, a subject is not induced to have a low blood sugar condition. In a certain embodiment, a subject is induced to have a low blood sugar condition, but is not induced to increase the blood sugar level.

According to the present invention, there is provided an antibody having a modified amino group. According to the present invention, the modification may reduce or eliminate (vanish) the binding affinity of the antibody for an antigen. This can be expected to reduce the functions of the antibody in non-target tissues, leading to a decrease in incidence of side effects and adverse events. The decrease in binding affinity is preferably greater. For example, the binding affinity of the modified antibody may be 30% or less, 25% or less, 20% or less, 15% or less, 10% or less, 5% or less, 4% or less, 3% or less, 2% or less, 1% or less, or 0.1% or less of the unmodified antibody. The binding affinity can be measured in, for example, serum or physiological saline. Thus, the antibody of the present invention can be inactivated by the modification. That is, the antibody of the present invention may be an inactivated antibody.

According to the present invention, the modification may be site-specific and environment-responsive. According to the present invention, the modification may be a reducing environment-responsive modification. This enables the antibody to be cleaved under a reducing environment in the brain parenchyma or a tumor tissue, whereby the binding affinity of the antibody for an antigen can be partially or completely recovered (reactivated). The recovery or reactivation is preferably greater. For example, the binding affinity of the recovered or reactivated antibody is 50% or more, 55% or more, 60% or more, 65% or more, 70% or more, 75% or more, 80% or more, 85% or more, 90% or more, or 95% or more of the binding affinity of the unmodified antibody. Thus, in the antibody of the present invention, the modification is environment-responsive, particularly, reducing environment-responsive, and can be cleaved in an environment-dependent manner to recover or reactivate the binding affinity of the antibody for an antigen. That is, the antibody of the present invention may be an environment-responsive reactivatable antibody.

According to the present invention, the modification for the antibody may further have a modification with a targeting molecule for a specific molecule, and may present the targeting molecule to the specific molecule. In this way, the antibody can be targeted at the specific molecule. Here, the specific molecule is immunologically distinguishably different from an antigen relevant to the antibody. That is, the antibody of the present invention may be a targeting antibody having ability to target a molecule immunologically distinguishable from an antigen.

According to the present invention, for example, the modification for the antibody may further have a modification with a targeting molecule for a specific molecule, and presents the targeting molecule to the specific molecule, and the modification reduces or eliminates the binding affinity of the antibody for an antigen. In particular, the binding affinity of the antibody of the present invention for an antigen of interest is reduced or eliminated by the modification. Therefore, ability to target a specific antigen is separately imparted to the modification, and the antibody is delivered to a target antigen site. According to the present invention, the modification is site-specific and environment-responsive, and in particular, the modification may be a reducing environment-responsive modification. Therefore, the antibody of the present invention may be, for example, an antibody having ability to target a molecule immunologically distinguishable from an antigen, the antibody being an inactivated antibody as well as an environment-responsive reactivatable antibody.

Reduction or elimination of the binding affinity of the antibody for an antigen may be caused by steric hindrance from a non-charged hydrophilic polymer block. Therefore, by making the non-charged hydrophilic polymer block bulky and/or increasing the modification ratio, the binding affinity of the antibody for an antigen can be more significantly reduced or eliminated.

The inactivated antibody can be attained by modifying an amino group of the antibody with a non-charged hydrophilic polymer block (for example, a polyethylene glycol block or polyoxazoline block). The non-charged hydrophilic polymer block can enhance the biocompatibility and the blood retainability of the antibody to improve the ability of the antibody to move to a target tissue. In addition, the non-charged hydrophilic polymer block has high water solubility, and can suppress the antibody from accessing an antigen of interest by extending around the antibody. In the present invention, a modification with a non-charged hydrophilic polymer block (for example, polyethylene glycol) capable of suppressing the binding affinity of the antibody for an original antigen of interest may be preferably used.

In the environment-responsive reactivatable antibody, a linker that is cleaved in an environment-responsive manner may be interposed between the antibody and the non-charged hydrophilic polymer block. The environment-responsive reactivatable antibody cleaves the linker in response to a specific tissue environment to separate the non-charged hydrophilic polymer block from the antibody, so that the antibody is liberated (released) to recover or reactivate the binding affinity of the antibody for an antigen. The linker that is cleaved in an environment-responsive manner has a reducing environment-cleavable bond (for example, a disulfide bond).

The targeting antibody may have a targeting molecule exposed at an end of a non-charged hydrophilic polymer block (for example, a polyethylene glycol block or polyoxazoline block) modifying an amino group of the antibody. The targeting antibody may have a targeting molecule targeting an inactivated antigen to a specific tissue.

According to the present invention, the antibody is, for example, a targeting antibody targeting a special environment where a disulfide bond is cleaved. The environment where a disulfide bond is cleaved may be, for example, a reducing environment (for example, brain parenchyma or tumor tissue), or a low-pH condition (for example, a physiologically possible low-pH condition). The antibody of the present invention may be, for example, an inactivated antibody as well as an environment-responsive reactivatable antibody in an environment other than the special environment where a disulfide bond is cleaved. In the present specification, such an antibody is referred to as an environment-targeting reactivatable antibody. For example, the pharmaceutical composition of the present invention comprises an environment-targeting reactivatable antibody. According to the present invention, the antibody may be a targeting antibody targeting the brain, the antibody being an inactivated antibody as well as a reducing environment-responsive reactivatable antibody. In the present specification, such an antibody is referred to as a brain-targeting reactivatable antibody. According to the present invention, the antibody may be a targeting antibody targeting a tumor, the antibody being an inactivated antibody as well as a reducing environment-responsive reactivatable antibody. In the present specification, such an antibody is referred to as a tumor-targeting reactivatable antibody.

According to the present invention, for example, a polyethylene glycol block terminally modified with a targeting molecule modifies an amino group of the antibody via an environment-responsive bond (for example, a reducing environment-responsive bond, for example, a disulfide bond). That is, the antibody may have an amino group modified with a linker in which non-charged hydrophilic polymer blocks (for example, polyethylene glycol blocks) terminally modified with a targeting molecule are linked via an environment-responsive bond (for example, a reducing environment-responsive bond, for example, a disulfide bond). According to the present invention, such an antibody can be provided.

According to the present invention, the antibody may bind to a cancer antigen. According to the present invention, the antibody may bind to any of immune checkpoint molecules. In a certain embodiment, examples of the antibody include LL1 (anti-CD74 antibody), LL2 or RFB4 (anti-CD22 antibody), vertuzumab (hA20, anti-CD20 antibody), rituximab (anti-CD20 antibody), obinutuzumab (GA101, anti-CD20 antibody), lambrolizumab (anti-PD-1 receptor antibody), nivolumab (anti-PD-1 receptor antibody), ipilimumab (anti-CTLA-4 antibody), RS7 (anti-epithelial glycoprotein-1 (EGP-1, also known as TROP-2) antibody), PAM4 or KC4 (both anti-mucin), MN-14 (anticancer fetal antigen (CEA, also known as CD66e or CEACAM5), MN-15 or MN-3 (anti-CEACAM6 antibody), Mu-9 (anti-colon specific antigen-p antibody), Immu 31 (anti-α-fetoprotein antibody), R1 (anti-IGF-1R antibody), A19 (anti-CD19 antibody), TAG-72 (for example, CC49 antibody), Tn, J591 or HuJ591 (anti-PSMA (prostate-specific membrane antigen) antibody, AB-PG1-XG1-026 (anti-PSMA dimeric antibody), D2/B (anti-PSMA antibody), G250 (anti-carbonic anhydrase IX Mab antibody), L243 (anti-HLA-DR antibody) alemtuzumab (anti-CD52 antibody), bevacizumab (anti-VEGF antibody), cetuximab (anti-EGFR antibody), gemtuzumab (anti-CD33 antibody), ibritumomab tiuxetan (anti-CD20 antibody); panitumumab (anti-EGFR antibody); tositumomab (anti-CD20 antibody); PAM4 (akatetraxetan (akaclivatuzumab), anti-mucin antibody), and trastuzumab (anti-ErbB2 antibody). These antibodies are known to those skilled in the art (see, for example, U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; and 7,642,239, and U.S. Patent Publication Nos. 20050271671; 20060193865; 20060210475; and 20070087001, Example sections of which are incorporated herein).

Specific known antibodies used include hPAM4 (U.S. Patent No. 7,282,567), hA20 (U.S. Patent No. 7,251,164), hA19 (U.S. Patent No. 7,109,304), hIMMU-31 (U.S. Patent No. 7,300,655), hLL1 (U.S. Patent No. 7,312,318), hLL2 (U.S. Patent No. 7,074,403), hMu-9 (U.S. Patent No. 7,387,773), hL243 (U.S. Patent No. 7,612,180), hMN-14 (U.S. Patent No. 6,676,924), hMN-15 (U.S. Patent No. 7,541,440), hR1 (U.S. Patent Application Publication No. 12/772,645), hRS7 (U.S. Patent No. 7,238,785), hMN-3 (U.S. Patent No. 7,541,440), AB-PG1-XG-026 (AB-PG1-XG1-026 (U.S. Patent Application Publication No. 11/983,372, represented as ATCC PTA-4405 and PTA-4406), and D2/B (International Publication No. WO 2009/130575). These cited patents or publications are incorporated herein by reference in their entirety. Without being limited, many antibodies for a variety of disease targets including tumor-related antigens have variable region sequences accumulated in ATCC and/or published, and are available for use in claimed methods and compositions. See, for example, U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572; 856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040, 6,451,310; 6,444,206; 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953; and 5,525,338. These documents are incorporated herein by reference in their entirety. These antibodies are merely illustrative, and a variety of other antibodies and hybridomas thereof are known to those skilled in the art. Those skilled in the art understand that in most cases, a sequence of an antibody or an antibody-secreted hybridoma for any of disease-related antigens can be obtained simply by searching ATCC, NCBI and/or USPTO for the antibody for the selected disease-related target of interest. An antigen-binding domain of a cloned antibody may be amplified, cleaved, linked to an expression vector, and genetically introduced into compatible host cells using a standard technique known to those skilled in the art, and may be used for protein production (see, for example, U.S. Patent Nos. 7,531,327; 7,537,930; 7,608,425; and 7,785,880, which are incorporated herein by reference in their entirety).

According to the present invention, examples of the target molecule (for example, antigen) include carbonic anhydrase IX, B7, CCCL19, CCCL21, CSAp, HER-2/neu, BrE3, CD1, CD1a, CD2, CD3, CD4, CD5, CD8, CD11A, CD14, CD15, CD16, CD18, CD19, CD20(for example, C2B8, hA20, 1F5 MAbs), CD21, CD22, CD23, CD25, CD29, CD30, CD32b, CD33, CD37, CD38, CD40, CD40L, CD44, CD45, CD46, CD47, CD52, CD54, CD55, CD59, CD64, CD67, CD70, CD74, CD79a, CD80, CD83, CD95, CD126, CD133, CD138, CD147, CD154, CEACAM5, CEACAM6, CTLA-4, α-fetoprotein (AFP), VEGF, fibronectin splicing variant, ED-B, fibronectin (for example, L19), EGP-1 (TROP-2), EGP-2 (for example, 17-1A), EGF receptor (ErbB1), ErbB2, ErbB3, H factor, FHL-1, Flt-3, folate receptor, Ga733, GRO-β, HMGB-1, hypoxia-inducible factor (HIF), HM1.24, HER-2/neu, histone H2B, histone H3, histone H4, insulin-like growth factor (ILGF), IFN-γ, IFN-α, IFN-β, IFN-λ, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, ganglioside, HCG, HLA-DR antigen binding to L243, CD66 antigen, i.e., CD66a-d, or combinations thereof, MAGE, mCRP, MCP-1, MIP-1A, MIP-1B, macrophage migration inhibition factor (MIF), MUC1, MUC2, MUC3, MUC4, MUC5ac, placental growth factor (PlGF), PSA (prostate-specific antigen), PSMA, PAM4 antigen, PD-1 receptor, PD-L1, NCA-95, NCA-90, A3, A33, Ep-CAM, KS-1, Le (y), mesothelin, S100, tenascin, TAC, Tn antigen, Thomas-Friedenreich antigen, tumor necrosis antigens, tumor neovascularization antigens, TNF-α, TRAIL receptors (R1 and R2), TROP-2, VEGFR, RANTES, T101, cancer stem cell antigens, complement factors, C3, C3a, C3b, C5a, C5, and oncogene products. The targeting molecule may be an antibody that binds to any of the above-mentioned target molecules, an antigen-binding fragment thereof, a ligand, an aptamer, or peptide.

In a certain embodiment, the antibody can bind specifically to an antigen. In a certain embodiment, the antibody may have a binding affinity (KD = k_{off}/kₒₙ) of 10⁻⁸ M or less, 10⁻⁹ M or less, 10⁻¹⁰ M or less 10⁻¹¹ M or less for an antigen. In a certain embodiment, the antibody may have a binding affinity of 10⁻¹⁵ M or more, 10⁻¹⁴ M or more, 10⁻¹³ M or more, 10⁻¹² M or more, 10⁻¹¹ M or more or 10⁻¹⁰ M or more for an antigen. In a certain embodiment, the antibody may have a binding affinity of 10⁻⁸ M to 10⁻¹⁵ M, 10⁻⁹ M to 10⁻¹⁴ M, 10⁻¹⁰ M to 10⁻¹³ M or 10⁻¹¹ M to 10⁻¹² M, or a binding affinity between any of the above-described upper limits and any of the above-described lower limits, for an antigen. In a certain embodiment, the modified antibody may have a binding affinity of 10⁻⁶ M or more, 10⁻⁵ M or more, 10⁻⁴ M or more, 10⁻³ M or more, 10⁻² M or more, or equal to or below the detection limit, for an antigen. In a certain embodiment, the modified antibody may have a binding affinity of 10⁻⁶ M to 10⁻¹ M, 10⁻⁴ M to 10⁻¹ M, 10⁻³ M to 10⁻¹ M or 10⁻² M to 10⁻¹ M, or equal to or below the detection limit, for an antigen. In a certain embodiment, the antibody may have the above-described antibody's binding affinity for an antigen, and the modified antibody may have the above-described modified antibody's binding affinity for an antigen.

According to the present invention, the targeting molecule may be a GLUT1 ligand. That is, according to the present invention, there is provided an antibody modified with a GLUT1 ligand, wherein the GLUT1 ligand, a non-charged hydrophilic polymer block, an environment-responsive bond and the antibody is linked in this order. The environment-responsive bond is cleaved under a reducing environment in the brain parenchyma or a tumor tissue. The environment-responsive bond may be stable in the blood. Therefore, even when administered to the blood, the antibody of the present invention maintains its existence form that is an antibody modified with a GLUT1 ligand, wherein the GLUT1 ligand, a non-charged hydrophilic polymer block, an environment-responsive bond and the antibody is linked in this order. When the antibody is incorporated in the brain parenchyma or a tumor tissue, the environment-responsive bond is cleaved, so that the non-charged hydrophilic polymer block is separated from the antibody. In the present invention, the modification is performed on the amino group of the antibody.

According to the present invention, there is provided an antibody in a complex form in which a GLUT1 ligand, a non-charged hydrophilic polymer block, an environment-responsive bond and the antibody is linked in this order. According to the present invention, there is provided a modified antibody having a structure of non-charged hydrophilic polymer block - environment responsive bond - antibody. Here, the symbol "-" represents a bond or a spacer, and means that an element written before the symbol and an element written after the symbol are bound, or linked via a spacer. The spacer has stable chemical properties in a living organism. In a certain embodiment of the present invention, the environment-responsive bond is a reducing environment-responsive bond, for example, a disulfide bond. In the present invention, the GLUT1 ligand may be glucose. The glucose is linked to the non-charged hydrophilic polymer block in a state of being able to bind to GLUT1.

According to the present invention, there is provided an antibody having an amino group modified with -C(O)-O-L₁-S-S-L₂- (non-charged hydrophilic polymer block), wherein L₁ is an optionally substituted lower alkylene (i.e., a substituted or unsubstituted lower alkylene), preferably ethylene, and L₂ is a bond (single bond), or a non-cleavable spacer (for example, a stable spacer). The non-charged hydrophilic polymer block may, but is not required to, be further modified with a targeting molecule. In this embodiment, L₂ may be L₃-O-C(O)-NH-L₄. When such an antibody is cleaved at a disulfide bond under a reducing environment, an unmodified antibody may be released. According to the present invention, the released unmodified antibody can recover the original binding affinity for an antigen.

According to the present invention, there is provided an antibody having an amino group modified with -C(O)-O-L₁-S-S-L₃-O-C(O)-NH-L₄- (non-charged hydrophilic polymer block), wherein L₁, L₃ and L₄ are each independently a bond or an optionally substituted lower alkylene. The antibody may be a reducing environment-responsive reactivatable antibody (for example, a brain-targeting reactivatable antibody). The non-charged hydrophilic polymer block may, but is not required to, be further modified with a targeting molecule. In the present invention, the targeting molecule may be a GLUT1 ligand. In the present invention, the GLUT1 ligand may be glucose. The glucose is linked to the non-charged hydrophilic polymer block in a state of being able to bind to GLUT1. When such an antibody is cleaved at a disulfide bond under a reducing environment, an unmodified antibody may be released. According to the present invention, the released unmodified antibody can recover the original binding affinity for an antigen.

The antibody is cleaved at a disulfide bond (-S-S-) in an environment-responsive manner, for example, under a reducing environment (for example, a reducing environment in the brain parenchyma or a tumor tissue) or under a low-pH condition (for example, a physiologically possible low-pH condition). When the disulfide bond is cleaved, the non-charged hydrophilic polymer block dissociates from the antibody, so that the antibody can recover the binding affinity for an antigen.

In a certain embodiment, the antibody may release an intact antibody (or an unmodified antibody or a naive antibody) by a chain reaction after cleavage of the disulfide bond. The term "intact antibody" means that the modified amino group returns to a -NH₂ group. The released intact antibody can recover the original binding affinity for an antigen.

In a certain embodiment, the binding affinity of the antibody for an antigen of interest is reduced or lost by modification with a non-charged hydrophilic polymer block. In a certain embodiment, the binding affinity of the antibody for an antigen of interest is reduced to 50% or less, 40% or less, 30% or less, 20% or less, 10% or less, 5% or less, 4% or less, 3% or less, 2% or less, 1% or less, 0.1% or less, or 0.01% or less by modification with a non-charged hydrophilic polymer block. When the binding affinity of the antibody is reduced or lost by modification with a non-charged hydrophilic polymer block, the antibody is inactive before cleavage of the environment-responsive, and therefore, the antibody can be prevented from functioning in areas other than a targeting site. This enables the function of the antibody to be recovered in a targeting site-specific manner.

The disulfide may bind to an antigen present in an environment where a disulfide bond is cleaved, for example, a reducing environment (for example, a reducing environment in the brain parenchyma or a tumor tissue) or under a low-pH condition (for example, a physiologically possible low-pH condition). The binding affinity (KD) of an unmodified antibody for an antigen may be, for example, 10⁻⁸ M or less, 10⁻⁹ M or less, 10⁻¹⁰ M or less, 10⁻¹¹ M or less, or 10⁻¹² M or less.

An antigen presents in an environment where a disulfide bond is cleaved, for example, a reducing environment (for example, a reducing environment in the brain parenchyma or a tumor tissue) or under a low-pH condition (for example, a physiologically possible low-pH condition) may be, for example, a cancer antigen, or an immune checkpoint molecule. The term "immune checkpoint molecule" means a molecule related to an immune checkpoint. The immune checkpoint molecule binds to a counterpart thereof (i.e., an immune checkpoint molecule as a binding partner) to activate the immune checkpoint, thereby suppressing the immunological action. The immune checkpoint molecule may be, for example, a molecule related to a PD-1-axis immune checkpoint. Examples of the molecule related to a PD-1-axis immune checkpoint include programmed cell death-1 (PD-1), cytotoxic T-lymphocyte-associated protein 4 (CTLA-4), T-cell immunoglobulin domain and mucin domain-3 (or TIM-3), lymphocyte activation gene 3 (LAG-3), and V-type immunoglobulin domain-containing suppressor of T-cell activation (VISTA). The immune checkpoints covered by the respective molecules are called a PD1-axis immune checkpoint, a CTLA-4-axis immune checkpoint, a TIM-3-axis immune checkpoint, a LAG-3-axis immune checkpoint, and a VISTA-axis immune checkpoint. An immune checkpoint inhibitor can bind to, for example, an immune checkpoint molecule or a counterpart thereof to inhibit the function of the immune checkpoint. For example, by inhibiting the binding of PD-1 and PD-L1 or PD-L2, the PD-1-axis immune checkpoint can be inhibited. By inhibiting the binding of CTLA-4 and CD80 or CD86, the CTLA-4-axis immune checkpoint can be inhibited. By inhibiting the binding of TIM-3 and galectin-9, the TIM-3-axis checkpoint can be inhibited. By inhibiting the binding of LAG-3 and a MHC class II molecule, the LAG-3-axis immune checkpoint can be inhibited. By inhibiting the binding of VISTA and VSIG-3/IGSF11, the VISTA-axis checkpoint can be inhibited. In this way, one or more immune checkpoints selected from the group consisting of a PD-1-axis immune checkpoint, a CTLA-4-axis immune checkpoint, a TIM-3-axis immune checkpoint, a Lag 3-axis immune checkpoint and a VISTA-axis immune checkpoint can be inhibited. The antibody inhibiting the binding of two proteins can bind to an immune checkpoint molecule or a counterpart thereof. For example, the antibody inhibiting the PD-1-axis immune checkpoint may be an antibody selected from the group consisting of an anti-PD-1 antibody, an anti-PD-L1 antibody and an anti-PD-L2 antibody (for example, nivolumab, pembrolizumab, avelumab, atezolizumab and durvalumab). The antibody inhibiting the CTLA-4-axis immune checkpoint may be an antibody selected from the group consisting of an anti-CDLA-4 antibody, an anti-CD80 antibody and an anti-CD86 antibody (for example, ipilimumab and tremelimumab). The antibody inhibiting the TIM-3-axis immune checkpoint may be an anti-TIM-3 antibody and an anti-galectin-9 antibody (for example, MGB453). The antibody inhibiting the VISTA-axis immune checkpoint may be an antibody selected from the group consisting of an anti-VISTA antibody and an anti-VSIG-3/IGSF11 antibody (for example, JNJ-61610588).

In a certain preferred embodiment, the antibody for use in the present invention binds to an immune checkpoint molecule expressed in immune cells (for example, PD-1, CTLA4, LAG-3, TIGIT, VISTA or TIM-3). In another preferred embodiment of the present invention, the antibody for use in the present invention binds to a counterpart of an immune checkpoint molecule expressed in immune cells (for example, PD-L1, PD-L2, CD80, CD86, galectin-9, MHC class II molecule, or VSIG-3/IGSF11).

The PD-1-axis immune checkpoint can be inhibited by blocking the binding of PD-1 and PD-L1. Alternatively, the PD-1-axis immune checkpoint can be inhibited by blocking PD-1 signaling.

Therefore, the antibody of the present invention may be an immune checkpoint inhibitor. The antibody of the present invention can bind to any of immune checkpoint molecules to block the binding between the molecule and an immune checkpoint molecule serving as a counterpart thereof. The antibody of the present invention may be a PD-1-axis immune checkpoint inhibitor. The antibody of the present invention may be, for example, an antibody capable of blocking the binding between PD-1 and PD-L1. The antibody of the present invention may be, for example, an antibody that binds to PD-1 (or anti-PD-1 antibody). The antibody of the present invention may be, for example, an antibody that binds to PD-L1 (or anti-PD-L1 antibody). The antibody of the present invention may be an anti-PD-L1 antibody capable of blocking the binding between PD-1 and PD-L1. The antibody of the present invention may be, for example, an anti-PD-1 antibody capable of blocking the binding between PD-1 and PD-L1. The term "blocking" mean that the binding is inhibited by 50% or more, 60% or more, 70% or more, 80% or more, 90% or more, or 95% or more.

The term "spacer stable in the blood flow" means a spacer which is stable enough to exist in the blood with stability over a time required for delivery to a target tissue (for example, the brain parenchyma or a tumor tissue) with the blood flow after administration. A bond selected from the group consisting of a carbon-carbon bond, an amide bond, a phosphodiester bond, an ester bond, an ether bond, an alkylene, a carbamate bond, a thiocarbamate bond, a thioester bond, a thioether bond, a disulfide bond, and a combination thereof may be a spacer that is stable in the blood flow. In the present invention, the spacer stable in the blood flow is pharmaceutically acceptable. Therefore, the non-charged hydrophilic polymer segment and the antibody may be linked via the spacer. The spacer stable in the blood flow is not limited, and is for example, a substituted or unsubstituted alkylene, or a substituted or unsubstituted heteroalkylene. Whether the spacer is stable in the blood flow can be determined by, for example, evaluating the stability of spacer in isolated blood, or physiological saline containing serum. Those skilled in the art can appropriately determine a time required for delivery to a target tissue (for example, the brain parenchyma or a tumor tissue) after administration. The time required for delivery to a target tissue (for example, the brain parenchyma or a tumor tissue) after administration may be, for example, 1 hour or more, 2 hours or more, 3 hours or more, 4 hours or more, 5 hours or more, 6 hour or more, 7 hours or more, 8 hours or more, 9 hours or more, 10 hours or more, 12 hours or more, 15 hours or more, 18 hours or more, 21 hours or more, 24 hours or more, 2 days or more, 3 days or more, 4 days or more, 5 days or more, 6 days or more, or 7 days or more. The time required for delivery to a target tissue (for example, the brain parenchyma or a tumor tissue) after administration may be, for example, 7 days or less, 6 days or less, 5 days or less, 4 days or less, 3 days or less, 2 days or less, or 1 day or less. The time required for movement to a target tissue (for example, the brain parenchyma or a tumor tissue) after administration may be, for example, 1 hour or more and 1 day or less.

According to the present invention, there is provided an antibody modified with a non-charged hydrophilic polymer segment, wherein the antibody can bind to an antigen, and the antibody is in a form modified with a non-charged hydrophilic polymer segment in the blood, and in a form dissociated from the non-charged hydrophilic polymer segment after delivery from the blood vessel to a special environment (for example, the brain parenchyma or a tumor tissue) where a disulfide bond is cleaved.

According to a certain embodiment of the present invention, the antibody passes through the vascular endothelial cells, and is dissociated from the non-charged hydrophilic polymer segment when entering a special environment where a disulfide bond is cleaved. For example, the antibody is dissociated from the non-charged hydrophilic polymer segment in a tumor tissue when entering the tumor tissue, or during transcytosis of BBB or in the brain parenchyma when entering the brain parenchyma. In this embodiment, the antibody is linked to the non-charged hydrophilic polymer segment by a reducing environment-responsive linker, and the relevant bond is cleaved under a reducing environment in a target tissue (for example, the brain parenchyma or a tumor tissue). In this embodiment, the reducing environment-responsive bond may be a disulfide bond.

According to a certain embodiment of the present invention, the antibody dissociated from the non-charged hydrophilic polymer segment has stronger binding affinity for an antigen than the antibody modified with the non-charged hydrophilic polymer segment.

According to a certain embodiment of the present invention, the antibody modified with the non-charged hydrophilic polymer segment has weaker binding affinity for an antigen than the antibody before modification (the unmodified antibody).

According to a certain embodiment of the present invention, the antibody modified with the non-charged hydrophilic polymer segment has weaker binding affinity for an antigen than the antibody before modification (unmodified antibody), and the antibody dissociated from the non-charged hydrophilic polymer segment has stronger binding affinity for an antigen than the antibody modified with the non-charged hydrophilic polymer segment.

According to the present invention, in a special environment where a disulfide bond is cleaved, for example, in the brain parenchyma or a tumor tissue, the antibody modified with the non-charged hydrophilic polymer segment is cleaved from the segment, and can recover the binding property of the antibody which has been reduced by the modification. Such an antibody may be useful because in a patient with, for example, a disease involving fragile vascular endothelial cells or causing fragility of the blood-brain barrier, the antibody can reach the relevant environment (for example, brain parenchyma or a tumor tissue) and recovers the binding property of the antibody in the environment (the brain parenchyma or a tumor tissue) without performing special manipulation (for example, blood sugar manipulation) or modifying the antibody with glucose. According to the present invention, the non-charged hydrophilic polymer segment may be modified with a GLUT1 ligand in this embodiment. The modification with a GLUT1 ligand is useful from the viewpoint that in a subject having a vascular endothelium (for example, a blood-brain barrier) expressing GLUT1, the relevant antibody is delivered into endosomes of vascular endothelial cells (for example, cerebrovascular endothelial cells) or a target tissue (for example, the brain parenchyma or a tumor tissue).

According to the present invention, there is provided an antibody modified with a non-charged hydrophilic polymer via a linker, wherein the non-charged hydrophilic polymer is modified with a GLUT1 ligand. The antibody can pass through the blood vessel of a tumor tissue, and enter the tumor tissue by an EPR effect or the like. Alternatively, the antibody can bind via a GLUT1 ligand to GLUT1 expressed on the lumen-side surfaces of vascular endothelial cells (for example, cerebrovascular endothelial cells). When glucose (or a GLUT1 ligand) is administered to a subject having reduced blood sugar, a substance bound to the lumen-side surfaces of the vascular endothelial cells (for example, cerebrovascular endothelial cells) of the subject is incorporated into the vascular endothelial cells by endocytosis, and at least a part of the substance is delivered by transcytosis into a special environment (for example, the brain parenchyma) where a disulfide bond is cleaved. GLUT 1 is expressed on the lumen-side surfaces of the vascular endothelial cells (for example, cerebrovascular endothelial cells) of the subject having reduced blood sugar, and the antibody of the present invention binds to GLUT 1 via a GLUT1 ligand. When glucose (or a GLUT1 ligand) is administered, the antibody is incorporated into the vascular endothelial cells by endocytosis, and at least a part of the antibody is delivered into the environment (for example, the brain parenchyma) by transcytosis. Here, the binding of GLUT1 and the antibody can be confirmed by an assay in which the binding of the isolated GLUT1 and the antibody in an *in vitro* experiment. Examples of the non-charged hydrophilic polymer include polyethylene (PEG) and polyoxazoline. Herein, the term "non-charged" means that charges are neutralized throughout the polymer segment. The non-charged hydrophilic polymer is biocompatible.

In a certain embodiment, the antibody of the present invention may, but is not required to, have a non-charged hydrophilic polymer segment modified with a ligand for a receptor in a target tissue (for example, the brain parenchyma or a tumor tissue) which is different from the GLUT1 ligand (a second ligand).

According to the present invention, in the antibody of the present invention, the linker can be linked to a side-chain amino group of a lysine residue of the antibody. The linkage may be preferably a covalent bond. In a certain embodiment, the lysine residue to which the linker is linked may be present in heavy chain and/or light chain variable regions of the antibody. In a certain embodiment, the lysine residue to which the linker is linked may be present in CDR regions of heavy chain and/or light chain variable regions of the antibody.

The modification with a targeting molecule (for example, a GLUT1 ligand) may be used for delivery from the blood flow to a special environment (for example, the brain parenchyma or a tumor tissue) where a disulfide bond is cleaved, and become unnecessary after delivary to the special environment (for example, the brain parenchyma or a tumor tissue) where a disulfide bond is cleaved. Therefore, after the antibody moves to a special environment (for example, the brain parenchyma or a tumor tissue) where a disulfide bond is cleaved, PEG modified with a targeting molecule (for example, a GLUT1 ligand) modifying the antibody may be separated from the antibody. According to the present invention, the modification of a side-chain amino group of a lysine residue of the antibody may deteriorate the binding property of the antibody depending on a modification intensity. Therefore, after the antibody moves to a special environment (for example, the brain parenchyma or a tumor tissue) where a disulfide bond is cleaved, PEG modified with a targeting molecule (for example, a GLUT1 ligand) modifying the antibody may be separated from the antibody. The targeting molecule (for example, a GLUT1 ligand) may modify PEG. The targeting molecule (for example, a GLUT1 ligand) may modify a carbon atom or an oxygen atom at an end of PEG.

The special environment where a disulfide bond is cleaved may be, for example, the brain parenchyma or a tumor tissue. For example, the brain parenchyma or a tumor tissue has a reducing environment. The reducing environment in the brain parenchyma or a tumor tissue is comparable in intensity to a 2 mM glutathione (GSH) aqueous solution. By incorporating into a linker a cleavage site that can be cleaved under a reducing environment, the linker can be configured to be cleaved after the antibody is delivered to the brain parenchyma or a tumor tissue. Such a linker is referred to as a linker cleavable under a reducing environment. In the present invention, an antibody is provided which is modified, via a linker cleavable under a reducing environment, with a non-charged hydrophilic polymer (for example, PEG) modified with a targeting molecule (for example, a GLUT1 ligand). Preferably, the antibody of the present invention can cleave a linker in the brain parenchyma or a tumor tissue which provides a reducing environment. The linker cleavable under a reducing environment may have, for example, a disulfide bond as a cleavage site. When the antibody and PEG modified with a targeting molecule (for example, a GLUT1 ligand) are linked using a linker cleavable under a reducing environment, the linker is cleaved after the antibody is delivered to the brain parenchyma or a tumor tissue, so that the antibody can be released in the brain parenchyma or a tumor tissue.

In another embodiment, the linker cleavable under a reducing environment can be configured to turn the side-chain amino group of the lysine residue of the antibody into an unsubstituted amino group by cleavage. For example, when (antibody-NH)-CO-O-C₂H₄-S-S-L₂- (non-charged hydrophilic polymer) {where L₂ is a bond, or a linker stable in the blood flow} is used as a linker, the side-chain amino group of the lysine residue of the antibody is recovered by the following reaction when the disulfide bond in the linker is cleaved. As a result, the modified antibody releases an unmodified antibody (intact antibody). Hereinafter, a mechanism is shown in which the inventive antibody having glucose (Gluc) as the GLUT1 ligand, polyethylene glycol (PEG) as the non-charged hydrophilic polymer, and a -C₂H₄-O-CO- group as L₂ is dissociated from a linker under a reducing environment to return to the original antibody. This mechanism also applies to a case where the GLUT1 ligand is other than glucose and the non-charged hydrophilic polymer is other than PEG.

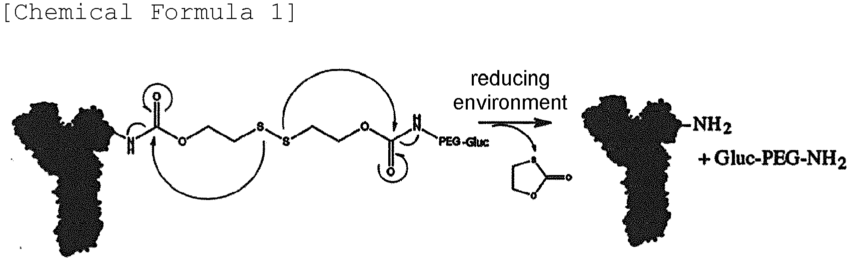

By being configured such that a linker moiety is dissociated from a side-chain amino group of a lysine residue of an antibody by cleavage, and the amino group returns to an unsubstituted amino group, the linker cleavable under a reducing environment can return the antibody to an unsubstituted state in a target tissue (for example, the brain parenchyma or a tumor tissue). It is pointed out that the binding property of the antibody may be deteriorated when the side-chain amino group of the lysine residue is modified. Even if the binding property of the antibody is deteriorated by modification of the side-chain amino group of the lysine residue, delivery of the antibody to a reducing environment in a target tissue (for example, the brain parenchyma or a tumor tissue) leads to removal of the modification of the side-chain amino group of the lysine residue of the antibody, resulting in restoration to an unsubstituted state, and this enables the antibody to recover the binding property thereof.

In a certain embodiment, the absolute value of the zeta potential of the modified antibody may be 10% or less, 5% or less, 4% or less, 3% or less, 2% or less or 1% or less of the absolute value of the zeta potential of the unmodified antibody. In a certain embodiment, the absolute value of the zeta potential of the modified antibody may be about 3% to 1% of the absolute value of the zeta potential of the unmodified antibody. The reduced absolute value of the zeta potential of the modified antibody is due to modification with a non-charged hydrophilic polymer block.

In a certain embodiment, the average hydrodynamic diameter (volume average) of the modified antibody is higher than that of the unmodified antibody by, for example, 10% or more, 20% or more, 30% or more, 40% or more, 50% or more, 60% or more, or 70% or more. In a certain embodiment, the average hydrodynamic diameter (volume average) of the modified antibody is higher than that of the unmodified antibody by 40% to 80%, or 50% to 70%.

In a certain embodiment, the antigen-binding ability of the modified antibody may be 20% or less, 15% or less, 10% or less, 9% or less, 8% or less, 7% or less, 6% or less, 5% or less, 4% or less, 3% or less, 2% or less, or 1% or less of the antigen-binding ability of the unmodified antibody.

In a preferred embodiment of the antibody of the present invention, the GLUT1 ligand may be glucose. In this embodiment, the glucose may be linked to PEG via the carbon atom at position 2, 3 or 6, and suitably interact with GLUT1.

In the antibody of the present invention, PEG may have a weight average molecular weight (Mw) of 2,000 to 12,000, for example, 5,000.

In the antibody of the present invention, 10 to 90%, 20 to 80%, 30 to 70% or 40 to 60% of the side-chain amino groups (primary amine) of the lysine residue may be modified with polyethylene glycol (PEG) via a linker. In the present specification, the proportion of PEG-modified amino groups in the side-chain amino groups of the lysine residue may be indicated by adding the relevant proportion (%) after the term "PEG".

The antibody of the present invention may be modified with both of PEG modified with a GLUT1 ligand and PEG which is not modified with a GLUT1 ligand. In the antibody of the present invention, for example, 10 to 100%, 30 to 80% or 40 to 60% of PEGs modifying the antibody may be modified with GLUT1. In the present specification, the proportion of PEGs modified with a GLUT1 ligand in the PEGs may be indicated by adding the relevant proportion (%) after the term "G" or "Gluc".

When the antigen relevant to an antibody is a surface antigen of a tumor in a target tissue (for example, the brain parenchyma) or a surface antigen of cells in a tumor tissue, the relevant antibody may be an antibody having antibody-dependent cellular cytotoxicity (ADCC activity) and/or complement-dependent cytotoxicity (CDC activity). The ADCC activity can be enhanced by, for example, using IgG1 as a subclass of the antibody. When the antigen relevant to an antibody is a surface antigen of a tumor in a target tissue (for example, the brain parenchyma) or a surface antigen of cells in a tumor tissue, the relevant antibody may be in a form of antibody-drug conjugate (ADC) with a cytotoxic agent. The antibody may be in a form of non-ADC.

In a certain embodiment, the antibody may be in a form of antibody-drug conjugate (ADC). In this embodiment, the linker modifies the antibody moiety. In a certain embodiment, the modification may have a sufficient length or size, and enclose the linker moiety of ADC to stabilize the linker against decomposition, or suppress decomposition of the linker by components in a living organism. The modification may have a sufficient length or size, and enclose the linker moiety and the drug moiety of ADC to reduce side effects caused by a situation in which the drug moiety of ADC is exposed and interacts with other components. As a non-limiting example, each of the numbers of linker moieties and drug moieties contained in ADC comprising a modified antibody moiety may be about 1 to 10, 2 to 8, 2 to 5, 2 to 4 or 3 to 4 per ADC. ADC comprising a modified antibody moiety may have an average hydrodynamic diameter (volume average) of 12 nm or more, 13 nm or more, 14 nm or more, 15 nm or more, 16 nm or more, 17 nm or more, about 18 nm, and/or 30 nm or less, 29 nm or less, 28 nm or less, 27 nm or less, 26 nm or less, or 25 nm or less, for example, 12 to 30 nm, or 15 to 25 nm. Without being limited, ADC may comprise one or more selected from the group consisting of, for example, gemtuzumab ozogamicin (MYLOTARG), ibritumomab tiuxetan (Severin), brentuximab vedotin (ADCetris), trastuzumab emtansine (KADCYLA), inotuzumab ozogamicin (Besponsa), moxetumomab pasudotox-tdfk (LUMOXITI), polatuzumab vedotin-Piiq (Polivy), trastuzumab deruxtecan (ENHERTS) and enfortumab vedotin (PADCEV).

The antibody of the present invention may be linked to an imaging agent. Examples of the imaging agent include biocompatible fluorescent dyes (for example, fluorescent dyes emitting fluorescence in the visible range or fluorescence in the near-infrared range) and emitting dyes (for example, luciferase), radioactive isotopes, ultrasonic probes, contrast agents for MRI, and contrast agents for CT. A conjugate of an imaging agent and an antibody can be appropriately prepared by those skilled in the art.

The antibody of the present invention may be linked to a physiologically active substance (for example, an enzyme or a nucleic acid). This enables a physiologically active substance to be delivered to a target tissue (for example, the bran parenchyma or a tumor tissue). A conjugate of a physiologically active substance and an antibody can be appropriately prepared by those skilled in the art.

According to the present invention, there is provided a pharmaceutical composition for treating or preventing a disease (for example, brain disease) in a target tissue, the pharmaceutical composition comprising the antibody of the present invention. Examples of the brain disease include a brain tumor. The brain tumors include, for example, a brain tumor selected from the group consisting of glioma, glioblastoma, primary central nervous system malignant lymphoma, meningioma, hypophysial adenoma, neurilemmoma and craniopharyngioma. Examples of the antibody include antibodies which bind to PD-L1 expressed in any of the above-mentioned tumors, and neutralize the activity of the PD-L1. Examples of the antibody include antibodies which bind to PD-1 expressed in the lymphocytes of any of the above-mentioned tumors, and neutralize the activity of the PD-1. Examples of the antibody include antibodies which bind to CTLA4 expressed in the lymphocytes of any of the above-mentioned tumors, and neutralize the activity of the CTLA4. In the present specification, the term "treatment of a disease" means prophylaxis of a disease and therapy of a disease. The term "prophylaxis of a disease" is used in the sense of including preventing the onset of a disease, delaying the onset, and reducing the incidence. The term "therapy of a disease" is used in the sense of including reduction in rate of worsening of a disease, delay of worsening, prevention of worsening, alleviation of a disease symptom, cure of a disease, and remission of a disease. According to the present invention, there is also provided a pharmaceutical composition for treating or preventing a disease (for example, cancer) in a target tissue, the pharmaceutical composition comprising the antibody of the present invention. Examples of the cancer include lung cancer (for example, lung squamous cell carcinoma, lung adenocarcinoma, small cell lung cancer and non-small cell lung cancer), head and neck cancer, hepatocyte cancer, renal cell cancer, bowel cancer, melanoma, bladder cancer, ovary cancer, stomach cancer, Merkel cell cancer, breast cancer (for example, triple-negative breast cancer), and Merkel cell cancer. Examples of the tumor include Hodgkin lymphoma.

The antibody or pharmaceutical composition of the present invention may be administered in accordance with a dosing regimen. The dosing regimen may comprise reducing the blood sugar level in a subject, subsequently inducing an increase in blood sugar level, and administering the antibody or pharmaceutical composition of the present invention so that a larger amount of the antibody is translocated to a target tissue (for example, the brain parenchyma) as compared to a case where an increase in blood sugar level is not induced. Here, the subject has a blood-brain barrier, or a functionally disrupted blood-brain barrier. The functionally disrupted blood-brain barrier drops a polymer containing an antibody to the brain parenchyma. Therefore, to a subject having a functionally disrupted blood-brain barrier, the modified antibody of the present invention can be administered without the dosing regimen.

In the dosing regimen according to the present invention, the composition can be administered to the subject simultaneously, successively or sequentially to induction of an increase in blood sugar level in the subject. The dosing regimen may, but is not required to, have an interval between administration of the composition to the subject and induction of an increase in blood sugar level in the subject. When the composition is administered simultaneously to induction of an increase in blood sugar level in the subject, the composition may be administered to the subject in a form mixed with a drug causing induction of an increase in blood sugar level, or may be administered in a form separate from a drug causing induction of an increase in blood sugar level in the subject. When the composition is administered successively or sequentially to induction of an increase in blood sugar level in the subject, the composition may be administered to the subject before or after induction of an increase in blood sugar level in the subject. Preferably, the composition can be administered to the subject before induction of an increase in blood sugar level in the subject. When an increase in blood sugar level is induced in the subject before administration of the composition to the subject, it is preferable to administer the composition to the subject within 1 hour, within 45 minutes, within 30 minutes, within 15 minutes or within 10 minutes after induction of an increase in blood sugar level in the subject. When an increase in blood sugar level is induced in the subject after administration of the composition to the subject, it is preferable to induce an increase in blood sugar level within 6 hours, within 4 hours, within 2 hours, within 1 hour, within 45 minutes, within 30 minutes, within 15 minutes or within 10 minutes after administration of the composition to the subject. Two or more cycles of the dosing regimen may be performed. The order relation between administration of glucose and administration of the sample can be determined by the timing of passage through the blood-brain barrier.

In another embodiment of the present invention, the GLUT1 ligand can be replaced by a molecule binding to a target antigen. The target antigen may be, for example, a membrane protein expressed on cell surfaces. This enables antibodies to be accumulated on specific cell surfaces. In an example, the antigen may be an antigen expressed in the cells of a target tissue (for example, cells of the brain). The molecule binding a target antigen, examples of which include proteins binding to a target antigen (for example, antibodies or cyclic proteins), aptamers binding to a target antigen, and lectins binding to a target antigen (when the target antigen has a sugar chain), can be appropriately selected or prepared, and used as a molecule binding to a target antigen of the present invention by those skilled in the art.

The pharmaceutical composition of the present invention may further comprise a pharmaceutically acceptable excipient in addition to the antibody of the present invention. The pharmaceutical composition of the present invention may be in any of various forms, for example, a solution (for example, an injection preparation), a dispersion, a suspension, a tablet, a pill, a powder and a suppository. In a preferred embodiment, the pharmaceutical composition of the present invention is an injection preparation, and may be administered parenterally (for example, intravenously, transdermally or intraperitoneally).

According to the present invention, there is provided a method comprising administering the antibody of the present invention to a subject. According to the present invention, there is provided a method for delivering an antibody to a target tissue (for example, the brain parenchyma or a tumor tissue) of a subject, the method comprising administering the antibody of the present invention or a pharmaceutical composition containing the antibody to the subject. The administration may be intravenous administration. In these methods, the antibody of the present invention may be administered in accordance with the dosing regimen according to the present invention. According to the present invention, there are provided a method for modifying an antibody, and a method for thereby obtaining the modified antibody of the present invention.

According to the present invention, there is provided use of the antibody of the present invention in the manufacture of the pharmaceutical composition of the present invention.

### Examples

### [Material and Method]

### Material

From Tokyo Chemical Industry Co., Ltd. (Tokyo, Japan), 1,2:3,4-di-O-isopropylidene-α-D-glucofuranoside (DIG) was purchased. From NOF CORPORATION (Tokyo, Japan), α-methoxy-ω-aminopolyethylene glycol (MeO-PEG-NH₂) (Mw of PEG is 5,500) was purchased. From Enamine Co., Ltd. (Tokyo), 2-[(2-[(4-nitrophenoxy)carbonyl]oxyethyl)disulfanyl]ethyl 4-nitrophenylcarbonate (NPC-(CH₂)₂-S-S-(CH₂)₂-NPC (NPC: p-nitrophenylcarbonate) was acquired. Alexa Fluor^{™} 647 MHS Ester (succinimidyl ester) was purchased from Thermo Fisher Scientific (MA, USA). Fluorescamine, reduced L-glutathione was acquired from Sigma-Aldrich Co. (St. Louis, MO). The organic solvent used in the present study was purchased from NIKKO HANSEN & CO., LTD. (Osaka, Japan). The organic solvent was purified by passing through two columns of neutral alumina. The cell membranes and nuclei of cell samples and sliced tissues were stained using CellMask^{™} Green Plasma Membrane Stain (Thermo Fisher Scientific, Catalog No. C37608) and Hoechst 33342 (Thermo Fisher Scientific, Catalog No. 62249).

### Cell line

Mouse glioblastoma cell line-GL261 tagged with luciferase and CT2A tagged with luciferase were acquired from JCRB Cell Bank (Osaka, Japan). GL261 and CT2A cells were maintained in RPMI 1640 medium (Gibco; Invitrogen) supplemented with 10% fetal bovine serum (Invitrogen), 100 U ml⁻¹ penicillin (Invitrogen) and 100 U ml⁻¹ streptomycin (Invitrogen). The cells were cultured in an 37 °C incubator (Thermo Fischer Scientific) under an atmosphere of 5% CO₂, 90% relative humidity.

### Antibody

Avelumab (trade name: BAVENCIO) which is a clinically approved human anti-PD-L1 antibody (aPD-L1) was commercially acquired from Merck & Co. The antibodies used in immunostaining were CD3 (BioLegend; Catalog No. 100205, clone: 17A2), CD4 (BioLegend; Catalog No. 100434, clone: GK1.5), CD45 (BD Biosciences; Catalog No. 553080, clone: 30-F11), CD8a (BD Biosciences; catalog No: 553035, clone: 53-6.7), FOXP3 (BD Biosciences; catalog No. 560408, clone: MF23), CD44 (BD Biosciences; catalog No. 553133, clone: IM7) and CD62L (BD Biosciences; catalog No. 553152, clone: MEL-14). Polychromatic flow cytometry was used after an appropriate adjustment was made. All the antibodies were used in accordance with written instructions from the manufacturers. The fluorescent dye conjugated to the antibody accurately matched the channel for the same fluorescent dye. After the staining, the cells were analyzed using a FlowJo software package. A recombinant mouse PDL1 protein was purchased from Abeam plc (Catalog No. ab130039). The secondary antibodies used in immunostaining and ELISA were goat anti-human IgG H&L (Abeam plc., Catalog No. ab97175) and goat anti-human IgG (H+L) (Thermo Fischer Scienttific, Catalog No. a-11013). Mouse

From Charles River Laboratories International, Inc. (Tokyo), C57BL/6J mice (female; 6 or 7 weeks of age) was purchased. All animal experiments were conducted on the basis of the ethical guidelines of Innovation Center of NanoMedicine which provides an experimental facility.

### Synthesis of MeO-PEG-NH-C(O)-O-(CH₂)₂-S-S-(CH₂)₂-NPC and DIG-PEG-NH-C(O)-O-(CH₂)₂-S-S-(CH₂)₂-NPC

First, DIG-PEG-NH₂ was synthesized as described in a previous report. Specifically, MeO-PEG-NH₂ (1.0 g, 0.167 mmol) or DIG-PEG-NH₂ (1.0 g, 0.167 mmol) dissolved in dichloromethane (DCM) was added to NPC-(CH₂)₂-S-S-(CH₂)₂-NPC (324 mg, 0.667 mmol) in an ice bath for 10 minutes. To the cold mixture, pyridine (54 µL, 0.667 mmol) was added dropwise with vigorous stirring. Thereafter, the reaction mixture was stirred overnight at room temperature, and subsequently dialyzed against DMSO using a membrane of MWCO 3.5 kDa. Finally, MeO-PEG-NH-C(O)-O-(CH₂)₂-S-S-(CH₂)₂-NPC and DIG-PEG-NH-C(O)-O-(CH₂)₂-S-S-(CH₂)₂-NPC were freeze-dried for subsequent use purposes.

To DIG-PEG-NH-C(O)-O-(CH₂)₂-S-S-(CH₂)₂-NPC (10.0 mg), TFA (0.5 mL, TFA/water = 4/1, v/v) was added. The mixture was stirred with vigorous stirring at room temperature for 30 minutes. Next, to the resulting solution, 10.0 mL of distilled water was slowly added in an ice bath. The obtained solution was dialyzed against water, 10 mM HCl and distilled water in this order. Finally, the purified solution was freeze-dried, and the ¹H-NMR of the obtained polymer was measured in D₂O at 25°C. As a result, the obtained polymer was a Gluc-PEG-NH-C(O)-O-(CH₂)₂-S-S-(CH₂)₂-NPC polymer where DIG was deprotected to form glucose.

As control polymers, MeO-PEG-NH-C(O)-O-(CH₂)₂-CH₂-CH₂-NPC and DIG-PEG-NH-C(O)-O-(CH₂)₂-CH₂-CH₂-CH₂-(CH₂)₂-NPC were prepared in the same manner as in the above-described synthesis step using NPC-(CH₂)₂-CH₂-CH₂-CH₂-(CH₂)₂-NPC that forms a non-cleavable bridge.

### Preparation of Gluc-S-aPD-Lls with various glucose densities

Solutions (1.0 mL) containing Gluc-PEG-(CH₂)₂-S-S-(CH₂)₂-NPC (10.0 mg mL⁻¹) and MeO-PEG-(CH₂)₂-S-S-(CH₂)₂-NPC (10.0 mg mL⁻¹) dissolved in phosphate buffer solutions (pH 8.4, 20 mM) at various supply ratios of 100%:0%, 50%:50%, 25%:75% and 0%:100%, respectively, were each mixed with aPD-Ll (1.0 mg), and the mixture was subjected to covalent binding overnight at room temperature. Next, the solution was purified with Vivaspin 6 (three times, cutoff MW: 30 kDa, 20 mM phosphate buffer having a pH of 7.4) to remove unreacted polymers, thereby obtaining Gluc-PEG-NH-C(O)-O-(CH₂)₂-S-S-(CH₂)₂-O-C(O)-NH-aPD-L1 (hereinafter, sometimes referred to as Gluc-S-aPD-Ll) which is an antibody complex with aPD-Ll linked to glucose through a disulfide bond. In this way, Gluc-S-aPD-Lls with various glucose densities (surface glucose densities) of 0%, 25%, 50% and 100% were prepared, and stored for subsequent use. The antibody can be modified with PEG-NH-C(O)-O-(CH₂)₂-S-S-(CH₂)₂-NPC (hereinafter, sometimes referred to as PEG-S-NPC) with an intensity causing the antibody to have significantly reduced binding affinity or lose binding affinity.

### Evaluation of properties of Gluc-S-aPD-Ll

The size distribution and the zeta potential of aPD-L1 and Gluc-S-aPD-Ll were evaluated by DLS measurement at 25°C in a 10 mM phosphate buffer solution at a pH of 7.4 using Zetasizer Nano ZS90 (Malvern Instruments Ltd., Worcestershire, England). For determining a PEG modification ratio, a solution of Fluorescamine in acetone (30.0 µL, 3.0 mg mL⁻¹) was mixed with Gluc-S-aPD-L1 (90 µL, 1.0 to 10.0 µg mL⁻¹), a florescence intensity at 666 nm was detected, and residual amine groups on the surface of aPD-Ll were counted with human serum albumin used as a control (60 amino groups per molecule). The gel permeation chromatography (GPC) spectra of aPD-Ll and Gluc-S-aPD-Ll incubated for various time periods with or without GSH (1.0 mM) were measured by size exclusion chromatography (Superose 6 Increase 10/300 columns, GE). For evaluating a change in molecular weight, an absorbance at 280 nm was recorded within 40 minutes. The circular dichroism spectra of aPD-Ll and Gluc-S-aPD-Ll incubated with or without GSH (1.0 mM) were measured at room temperature using a CD spectrometer (JASCO J-815).

### Reducing environment-responsive recovery of activation (reactivation) of Gluc-S-aPD-Ll

For evaluating the binding affinity between each of aPD-Ll and Gluc-S-aPD-Ll and the PD-L1 protein, an ELISA method was developed in which a recombinant mouse PD-L1 protein is coated on the bottom surface of a 96-well plate, and reacted with a test antibody (aPD-L1 or Gluc-S-aPD-Ll) and then with a HRP-labeled secondary antibody for the test antibody, and an absorbance at 450 nm is recorded to determine the amount of specific binding of the antibody to an antigen. Each of aPD-Ll and Gluc-S-aPD-Ll was preincubated for various time periods with or without GSH (1.0 mM) to cleave the disulfide bond, and then reacted with the PD-L1 protein, and the activation recovery ratio was calculated using the above-described system.

For observing the binding of each of aPD-Ll and Gluc-S-aPD-Ll and the PD-L1 protein expressed on the tumor cell surfaces, the cell membranes of GL261 tumor cells were stained with CellMask^{™} Green Plasma Membrane Stain, aPD-Ll and Gluc-S-aPD-Ll treated with or without GSH (1.0 mM) were incubated together with GL261 cells for 1 hour, and finally, the binding affinity of the antibody was evaluated using a confocal scanning laser microscope (LSM 880, Zeiss).

For quantitatively measuring the amount of binding of each of aPD-Ll and Gluc-S-aPD-Ll and the PD-L1 protein, GL261 tumor cells were incubated for 1 hour together with aPD-Ll and Gluc-S-aPD-Ll treated with or without GSH (1.0 mM), and washing was performed three times. Next, an APC-labeled anti-mouse PD-L1 antibody was incubated together with GL261 cells for 1 hour, analysis was then performed using the FlowJo software package, and the residual PD-L1 protein was measured.

### Pharmacokinetics in vivo and distribution in living organism

All animal experiments were conducted on the basis of the ethical guidelines of Innovation Center of NanoMedicine. In this Example, manipulation of blood sugar level was not performed in any of the experiments. For examining the pharmacokinetics, aPD-Lls and Gluc-S-aPD-Lls with various glucose densities were administered to healthy BALB/c mice through the tail vein at a dose of 1.0 mg kg⁻¹. A blood sample was collected 1 hour, 4 hours, 12 hours and 24 hours after the administration. The blood sample was extracted, and treated with or without GSH (10 mM). Thereafter, the concentration of aPD-Ll was determined using the ELISA protocol designed as described above.

For demonstrating a physiological distribution, Alexa 647-labeled aPD-Ll and Alexa 647-labeled Gluc-S-aPD-Lls (1.0 mg kg⁻¹) with various glucose densities were intravenously administered to C57BL/6J mice having an orthotopic GL261 tumor, and after 1 hour, 4 hours, 12 hours and 24 hours, the mice were sacrificed for extraction of the heart, the liver, the spleen, the lung, the kidney, the brain, and the tumor. Next, the tissues thereof were washed with D-PBS (-), an excessive washing solution was removed, the weight was then measured, and the tissues were homogenized with 600 µL of a cell lysis buffer solution. Finally, the biological distributions of aPD-Ll and Gluc-S-aPD-Ll were quantified by fluorescent measurement using an Infinite M1000 PRO spectrophotometer (Tecan Group Ltd., Mannedorf, Switzerland).

### In vivo observation of reducing environment-responsive cleavage of Gluc-PEG chain

Alexa 647-labeled aPD-Ll, Alexa 647-labeled Gluc-25-C-1PD-L1 and Alexa 647-labeled Gluc25-S-aPD-L1 were intravenously administered to mice having an orthotopic GL261 tumor. After 24 hours, the mice were sacrificed, and the tumor tissue was extracted. Thereafter, the tumor was cut into a 10.0 µm section, which was fixed with cold acetone, washed with PBS, and blocked with 5% BSA at room temperature for 1 hour. Thereafter, the section was incubated overnight at 4°C with Alexa 488-labeled anti-human IgG (H+L) (Thermo Fisher Scientific, Catalog No. A-11013), followed by CLSM observation.

### In vivo therapeutic effect on orthotopic brain tumor

GL261-luc or CT2A-luc cells expressing luciferase (luc) was intracerebrally inoculated to C57BL/6J mice at 1.0 mm front of the bregma, 2.0 mm right from the bregma and 3.0 mm deep from the bregma in the skull (1.0 × 10⁵ cells per infusion in an amount of 2 µL). The day on which the cells were infused into the mice was defined as Day 0. When tumor nodules were generated, the mice (n = 5) were randomized, and the preparations were administered by single tail vein injection (1.5 mg kg⁻¹ for aPD-Ll). Luciferin was injected in an amount of 150 mg kg⁻¹, and bioluminescent signals from the GL261-luc or CT2A-luc tumor were observed with an *in vivo* imaging system (IVIS). The survival durations of the mice were tracked, and whether the duration was significantly extended was determined by a log-rank test.

For a tumor re-challenge test for confirming establishment of memory immunity, C57BL/7J mice received 1.0 × 10⁵ GL261 cells first and 90 days after subsequent treatment with Gluc25-S-aPD-L1. Thereafter, bioluminescent signals were monitored, and survival durations were recorded.

### Analysis of T-cells and detection of INFγ

The plasma level of INFγ was measured using an INFγ ELISA kit (BioLegend; Catalog No. 430804). The peripheral blood was collected on 3 days after the injection of aPD-Ll, GlucO-S-aPD-Ll and Gluc25-S-aPD-L1, and centrifuged at 500 g for 10 minutes. The supernatant was separated, and stored at -80°C until analysis. The sample was diluted with an ELISA assay buffer in accordance with written instructions from the manufacturer, followed by analysis with the ELISA kit.

For analysis of T-cells, the tumor tissue was cut and removed 3 days after the injection of aPD-Ll, GlucO-S-aPD-Ll and Gluc25-S-aPD-L1, and dissociated with BD Horizon^{™} Dri Tissue & Tumour Dissociation Reagent. Subsequently, the tumor cells were stained with an anti-CD3 antibody, an anti-CD8 antibody, an anti-CD4 antibody, an anti-CD 45 antibody or an anti-Foxp3 antibody for 30 minutes. Staining with Foxp3 was performed in accordance with the intracellular staining protocol from Biolegend. Thereafter, the stained cells were subjected to flow cytometry for analysis of CD8 and Foxp3 cell populations (2 × 10⁴ cells were collected for analysis).

After 60 days, spleen cells were extracted from mice treated with physiological saline and Gluc25-S-aPD-L1 and stained with an anti-CD8 antibody, an anti-CD44 antibody and anti-CD62L antibody for analyzing memory T-cells. Thereafter, the stained cells were analyzed by flow cytometry (2 × 10⁴ cells were collected for the analysis).

### Analysis of CD45⁺ subset and detection of increased inflammation of normal organ

Unmodified aPD-Ll and Gluc25-S-aPD-L1 were intravenously administered to healthy mice (n = 8) at a dose of 10 mg kg⁻¹ three times, once a week. Five days after the injection, the lung, the liver and the kidney were extracted, and CD45⁺ cells were analyzed. On the other hand, the tissue was cut into 10.0 µm section, which was fixed with cold acetone, washed with PBS, and blocked with 5% BSA at room temperature for 1 hour. Thereafter, the section was incubated overnight at 4°C with Alexa 488-labeled anti-human IgG (H+L) (Thermo Fisher Scientific, Catalog No. A-11013), followed by CLSM observation.

The TNF-α, IL-6 and IL-1β levels of the tissues were measured using an ELISA kit (manufactured by Thermo Fischer Scientific, Catalog No. BMS607-3, KMC0061, and BMS6002). The tissues comprising the lung, the liver, and the kidney were subjected to various treatments in accordance with written instructions, then homogenized, and centrifuged at 500 g for 10 minutes. The supernatant was dispensed, and stored at -80°C until analysis. The sample was diluted with an ELISA assay buffer in accordance with instructions from the manufacturer, followed by analysis with the ELISA kit.

Alexa 647-labeled aPD-Ll and Alexa 647-labeled Gluc25-S-aPD-L1 were intravenously administered to mice. After 24 hours, the mice were sacrificed, and the lung, the liver, and the kidney were extracted. Thereafter, the tissue was cut into a 10.0 µm section, which was fixed with cold acetone, washed with PBS, and subsequently blocked with 5% BSA at room temperature for 1 hour. Thereafter, the section was incubated overnight at 4°C with Alexa 488-labeled anti-human IgG (H+L) (Thermo Fisher Scientific, Catalog No. A-11013), followed by CLSM observation.

### Statistics

Results are all shown in terms of an average ± s.d. or an average ± SEM. Statistical analysis was performed using GraphPad Prism (8.0). For statistical analysis of the survival duration, a log-rank test was conducted, and for other statistical analyses, unidirectional variance analysis (ANOVA), and then a Tukey's honestly significant difference (HSD) post-hoc test were conducted. The difference between the experimental group and the control group was considered to be statistically significant at P < 0.05. *P < 0.05; **P < 0.01; and ***P < 0.001.

### [Results]

### Preparation and property evaluation of reducing environment-responsive Gluc-S-aPD-Ll

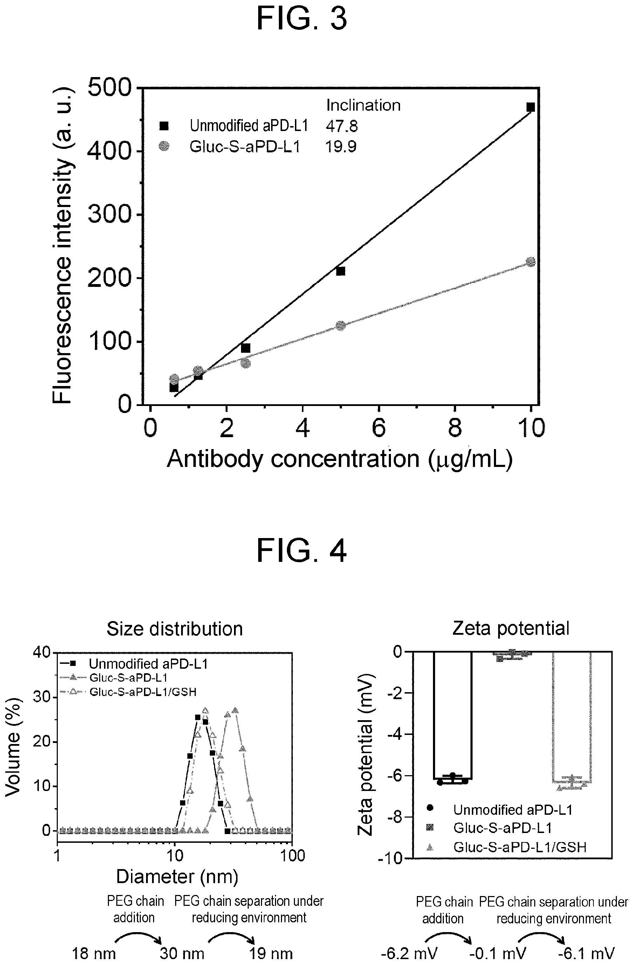

For constructing Gluc-S-aPD-Ll shown in Figure 1, first, a reducing environment-responsive PEG chain (DIG-PEG-S-NPC or PEG-S-NPC) was synthesized by covalent conjugation of NPC-(CH₂)₂-S-S-(CH₂)₂-NPC with one-side modification controlled by an excessive amount of DIG-PEG-NH₂ or α-CH₃O-PEG-NH₂ and DIG-terminated polyethylene glycol (DIG-PEG-NH₂) or α-methoxy-ω-aminopolyethylene glycol (α-MeO-PEG-NH₂), and DIG was then deprotected so as to form glucose. A characteristic peak of the polymer was identified in a ¹H nuclear magnetic resonance spectrum. Next, a series of Gluc-S-NPCs having different glucose concentrations (0, 25, 50, 100 mol%) were prepared by adjusting the supply ratio between Gluc-PEG-S-NPC and PEG-S-NPC, and then reacted with aPD-Ll amino groups by a covalent binding method. Thereafter, residue amino groups of aPD-Ll were detected with Fluorescamine used as a probe, and the result showed that about 60% of amino groups were modified (Figure 3).

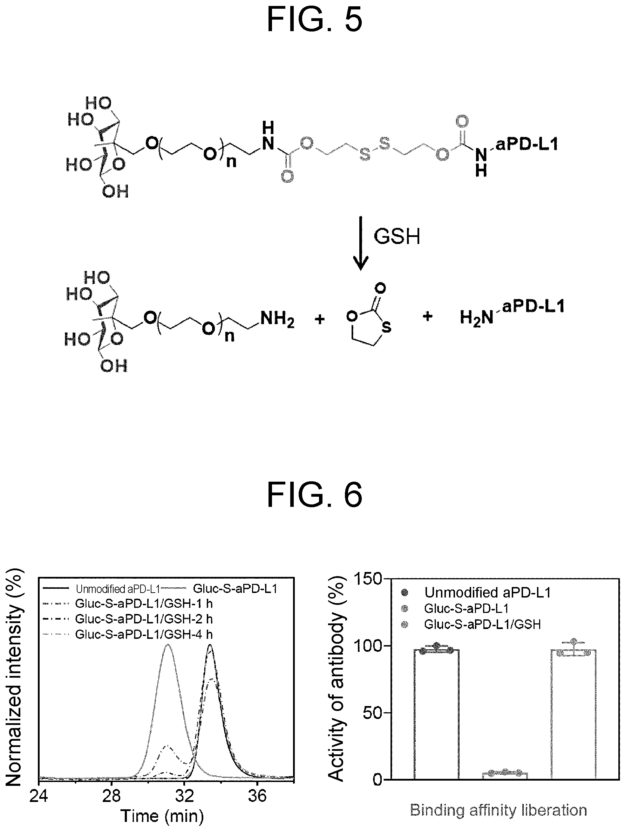

Further, for confirming modification of the antibody with a PEG chain and desorption of the PEG chain from the antibody, first, dynamic light scattering (DLS) analysis was performed to evaluate the size distribution and the zeta potential. As shown in the left panel of Figure 4, the hydrodynamic diameter of Gluc-S-aPD-Ll slightly increased, but was found to evidently decrease like unmodified aPD-Ll through PEG chain cleavage after glutathione (GSH) treatment. The volume average particle diameter of unmodified aPD-Ll was 10.2 nm, and the volume average particle diameter of Gluc-S-aPD-Ll was 18.8 nm. Further, the zeta potential of unmodified aPD-Ll was - 6.13 mV, and the zeta potential of Gluc-S-aPD-Ll was - 0.16 mV, whereas by the glutathione (GSH) treatment, the PEG chain was disrobed from the antibody to obtain a negative charge of -6.07 mV, which is substantially equal to the zeta potential of unmodified aPD-Ll (right panel of Figure 4). This demonstrated that as shown in Figure 5, aPD-Ll had an increased size and a raised zeta potential when modified with a glucose-modified PEG chain at an amino group in the amino acid side chain to form Gluc-S-aPD-Ll. It was indicated that under a reducing environment (for example, in the presence of GSH), Gluc-S-aPD-Ll underwent cleavage of the disulfide bond in the PEG chain, and accordingly returned to aPD-Ll in an unmodified state.

Further, by gel permeation chromatography (GPC), it was demonstrated that the molecular weight of Gluc-S-aPD-L1 evidently increased after PEGylation treatment. On the other hand, the peak related to Gluc-S-aPD-Ll was found to gradually return to that of original aPD-Ll over time under a reducing environment (Figure 6), leading to further clarification of a reducing environment-responsive PEG chain cleavage behavior. Further, the secondary structure of aPD-Ll after introduction and cleavage of a PEG chain was analyzed using a circular dichroism spectrum²⁴. Regardless of whether GSH treatment was performed or not, Gluc-S-aPD-Ll exhibited a β-sheet absorbance which was not significantly different from that of unmodified aPD-Ll, and this indicated that Gluc-S-aPD-Ll having a well conserved structure maintained a physiological function for PD-L1 even after cleavage of the PEG chain²⁵. Aside from the secondary structure, it was also confirmed that a physiological function was maintained because the surface structure of aPD-Ll free of residues occupying amine groups of lysine was conserved even after responsive cleavage of the PEG chain (Figure 5).

For examining the binding affinity of Gluc-S-aPD-Ll for a ligand, the capability of Gluc-S-aPD-Ll to bind specifically to a ligand was quantitatively determined by an enzyme-linked immunosorbent assay (ELISA)²⁶. As shown in the right part of Figure 6, Gluc-S-aPD-Ll did not significantly bind to PD-L1 in ELISA. On the other hand, when Gluc-S-aPD-Ll was exposed to a reducing environment (1.0 mM GSH, pH 7.4), the amount of binding of the antibody significantly increased in a time-dependent manner (substantially reaching a plateau after incubation for 2 hours or more), and this indicated that under a reducing environment, the disulfide bond of Gluc-S-aPD-Ll was cleaved, so that unmodified aPD-Ll was released ²⁷. Further, aPD-Lls having various PEG chain densities were prepared, and the binding affinity of the aPD-Lls were evaluated, and the results showed that the capability to bind to PD-L1 partially remained when the proportion of modified amine groups on the aPD-Ll surface was low.

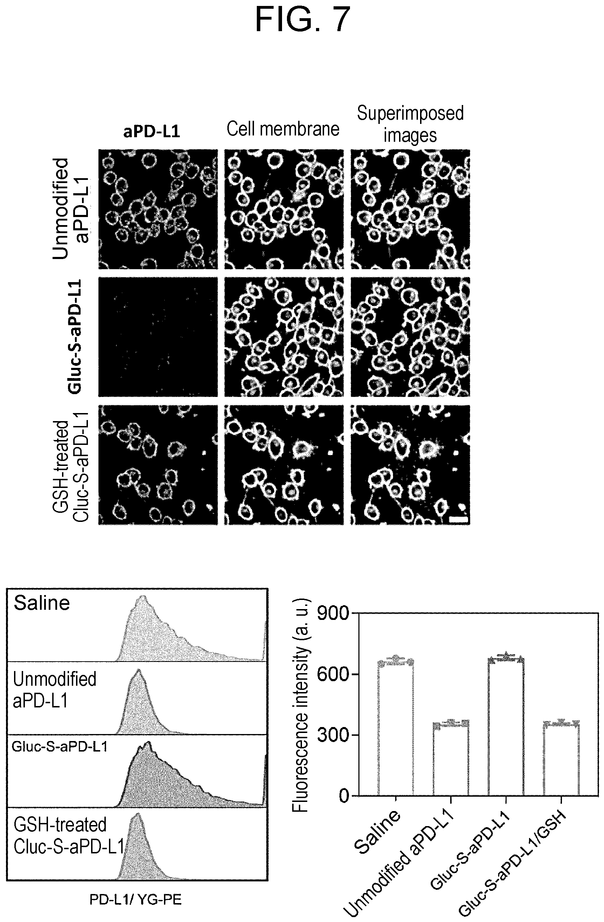

Since PD-L1 was expressed mainly on the membranes of tumor cells²⁸, the binding of Gluc-S-aPD-Ll specifically to GL261 glioblastoma cells was evaluated. As a positive control, unmodified aPD-Ll favorably colocalized with tumor cell membranes after incubation for 1.0 hour, but red fluorescence recognized after incubation of Gluc-S-aPD-Ll and tumor cells was ignorable (Figure 7). It is thought that his is because in Gluc-S-aPD-Ll, the PD-L1-binding ability of the antibody is significantly impaired due to modification with a PEG chain. On the other hand, Gluc-S-aPD-Ll treated under a reducing environment evidently recovered binding affinity for PD-L1 because the amount of unmodified aPD-Ll increased due to efficient cleavage of the PEG chain from the antibody (Figure 6 and Figure 7). This was also demonstrated by quantitatively measuring the expression of PD-L1 in GL261 cells by flow cytometric analysis. From the above results, it was confirmed that although the binding affinity of Gluc-S-aPD-Ll was significantly impaired due to PEG modification, Gluc-S-aPD-Ll recovered the binding capability due to cleavage of the PEG chain and release of unmodified aPD-Ll under a reducing environment. The brain parenchyma is known to provide a reducing environment in the body, and it is considered that Gluc-S-aPD-Ll recovered binding affinity in a brain parenchyma-specific manner to enable brain ICB therapy that is specific to a site in the brain (in particular, the antibody is inactive in other organs).

### Therapeutic effect of Gluc-S-aPD-Ll in vivo

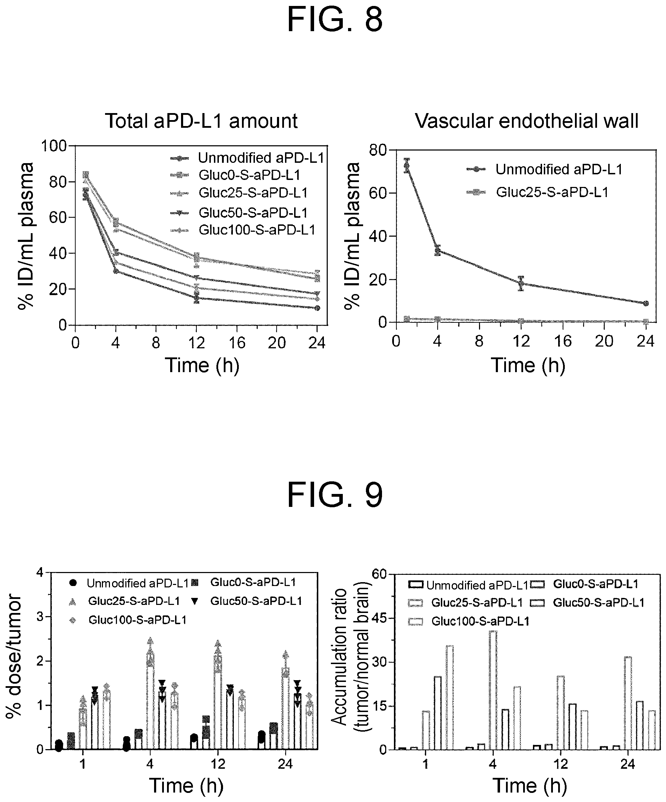

One of causes of failure of conventional glioblastoma (GBM) therapy is low efficiency of delivery of a therapeutic drug to the brain due to the presence of a blood-brain barrier (BBB)^{2,3}. Thus, promotion of selective accumulation of the ICB antibody in the GBM site may be important for improvement of a therapeutic index. For examining the capability of transportation of Gluc-S-aPD-Ll through BBB, first, the *in vivo* pharmacokinetics of Gluc-S-aPD-Ll was evaluated. As shown in the left panel of Figure 8, Gluc-S-aPD-Lls having various glucose densities had a significantly longer half-life (t_{1/2}**β** = 6.1 to 10.3 hours) over unmodified aPD-Ll (t_{1/2}**β** = 5.2 hours), and this is possibly because PEG shell coating of the core enhanced stability in the blood, and suppressed distribution to normal organs via off-target binding²³. Further, introduction of glucose at a higher density caused relatively short blood circulation because interaction with a GLUT family expressed in the liver is enhanced^{29,30}. It is indicated that regulation of the density at which glucose is introduced into aPD-Ll is very beneficial for extending circulation for enhancing accumulation of a tumor. Next, the capability of active transportation of Gluc-S-aPD-Ll through BBB was tested using an orthotopic GBM model. First, aPD-Ll was labeled with a fluorescent dye, and then modified with each of Gluc-PEG-S-NPC and PEG-S-NPC. Unmodified aPD-Ll and Gluc-S-aPD-Lls having different modification glucose densities were intravenously injected at equivalent aPD-Ll doses to C57B+/6J mice having an orthotopic GBM tumor, and main organs were extracted in 24 hours after the injection for fluorescent analysis. Apparently, unmodified aPD-Ll and control GlucO-S-aPD-Ll having no active targeting capability were accumulated in the tumor in the brain in 24 hours, but in a small amount. The amount of accumulation of Gluc25-S-aPD-L1 was the highest with the amount being 2.2% ID g⁻¹, and was about 6.1 times that in the GlucO-S-aPD-Ll and about 18.0 times that in the unmodified aPD-Ll group after initial 4 hours (left panel of Figure 9). This indicates that the capability of active transportation of the antibody through BBB was enhanced by glucose modification³¹⁻³³. On the other hand, Gluc-S-aPD-Ll is also accumulated in the liver, the spleen, the kidney and the lung, and this is correlated with the fact that aPD-Ll into which glucose is introduced at a high density is easily incorporated into the liver, and the levels of Gluc50-S-aPD-L1 and Cluc100-S-aPD-Ll in the circulated blood decrease. These phenomena may be caused by various GLUTs present in intravascular compartments of the liver, and indicate that an appropriate glucose density on the surface is a key for overcoming the liver barrier and enhancing tumor accumulation³¹. More interestingly, due to rapid delivery associated with an active transportation mechanism in which GLUC25-S-PD-L1 passes through BBB, selective accumulation in the brain tumor site occurred, and the tumor/brain ratio increased to a maximum of 45 (right panel of Figure 9). This indicates that the technique of the present invention is effective in immune checkpoint blockade (ICB) therapy in the brain. In the present experiment, special blood sugar manipulation (in particular, blood sugar manipulation such that the antibody is administered simultaneously to manipulation for increasing the blood sugar level increasing after fasting). In a subject with a brain tumor, BBB is broken, so that the drug can reach the brain tumor.

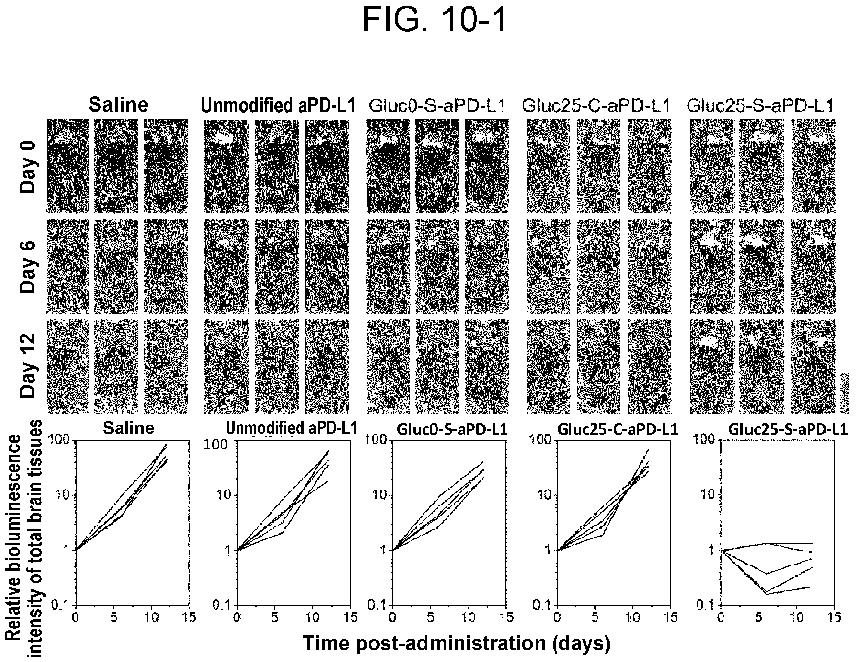

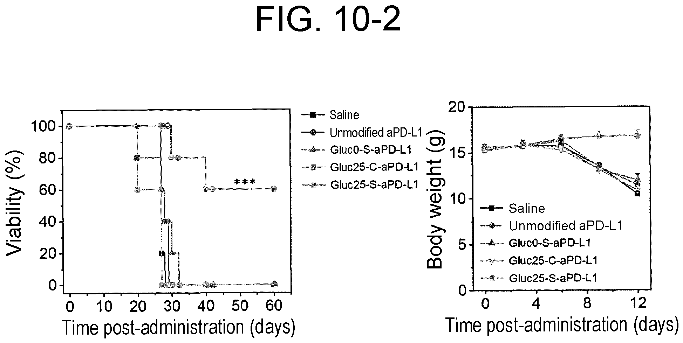

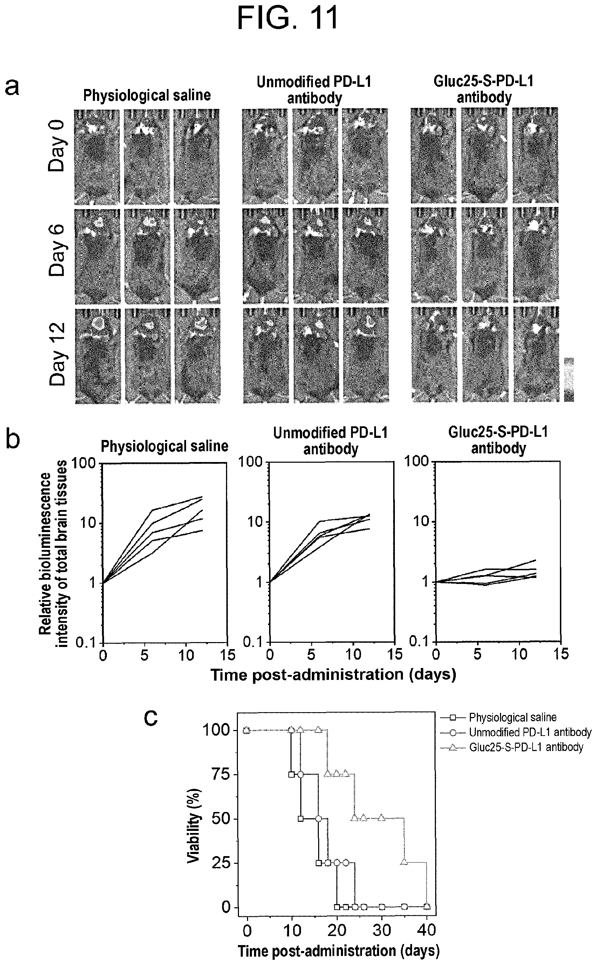

For examining the therapeutic effect of Gluc25-S-aPD-Ll on an orthotopic brain tumor, a GL261 cell line was inoculated into the right-side skull of the brain. Thereafter, physiological saline, aPD-Ll, GlucO-S-aPD-Ll and Gluc25-S-aPD-L1 were administered in a single dose to mice with a GL261 tumor. On the other hand, inactivated (non-cleavable) Gluc25-C-aPD-L1 was applied as a control. The onset of the brain tumor was tracked by bioluminescence from GL261 cells in the total brain. As shown in Figure 10-1, bioluminescence decreased after 6 days for mice treated with Gluc25-S-aPD-L1, and GL261 cells were detected only at an ignorable level in the brain site for two of five mice. This indicates a strong immune response. In contrast, for mice receiving unmodified aPD-Ll and GlucO-S-aPD-Ll, there was only a small therapeutic effect on the GL261 tumor. Thus, the therapeutic effect depended on the amount of accumulation of the antibody in the brain. Further, the GL261 tumor in mice receiving inactivated (non-cleavable) Gluc25-C-aPD-Ll also exhibited a potent proliferation profile, and this is because inactivated (non-cleavable) Gluc25-C-aPD-L1 does not release aPD-Ll in response to reduction, indicating that the presence of a reducing environment and a reducing environment-responsive linker plays an important role in brain-directive ICB therapy according to the present invention (lower five panels of Figure 10-1). For mice receiving Gluc25-S-aPD-L1, the viability after 90 days was about 60%, but any of the mice did not survive for 32 days or more, and an evident body weight loss was recognized in all other control groups (Figure 10-2). In view of the potent anticancer effect against GL261, Gluc25-S-aPD-L1 was applied to therapy for a CT-2A cell line. The CT-2A cell line exhibits a low level of expression of PTEN³⁴, accurately reflects some characteristics of clinical glioblastoma, including intratumor non-homogeneity, and has radiation resistance and chemotherapy resistance due to the presence of a high proportion of cancer stem cells^{34,35}. For mice with a CT-2A tumor in the physiological saline administration group, the survival duration was less than 20 days, and the tumor was actively proliferated (Figure 11). The same applied to the unmodified aPD-Ll administration group. On the other hand, for mice receiving Gluc25-S-aPD-L1, growth of the tumor was markedly delayed, and the survival duration was extended (Figure 11). This indicates that both active delivery of the ICB antibody through BBB and reactivatable ICB therapy enhance the therapeutic effect against aggressive glioblastoma via a sufficient and strong antitumor immune response.

### Cleavage of PEG chain of Gluc-S-aPD-Ll in living organism

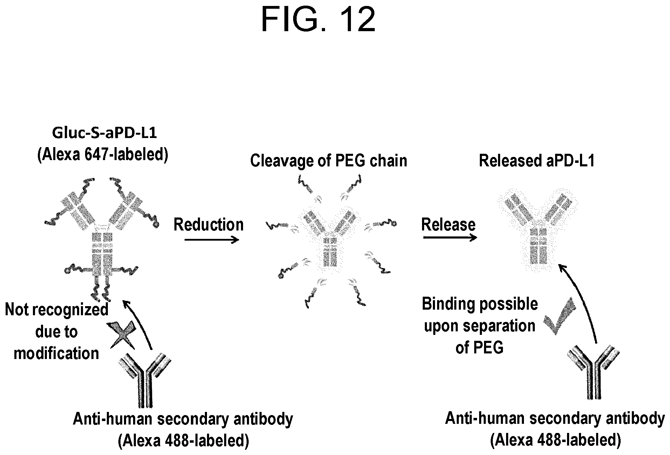

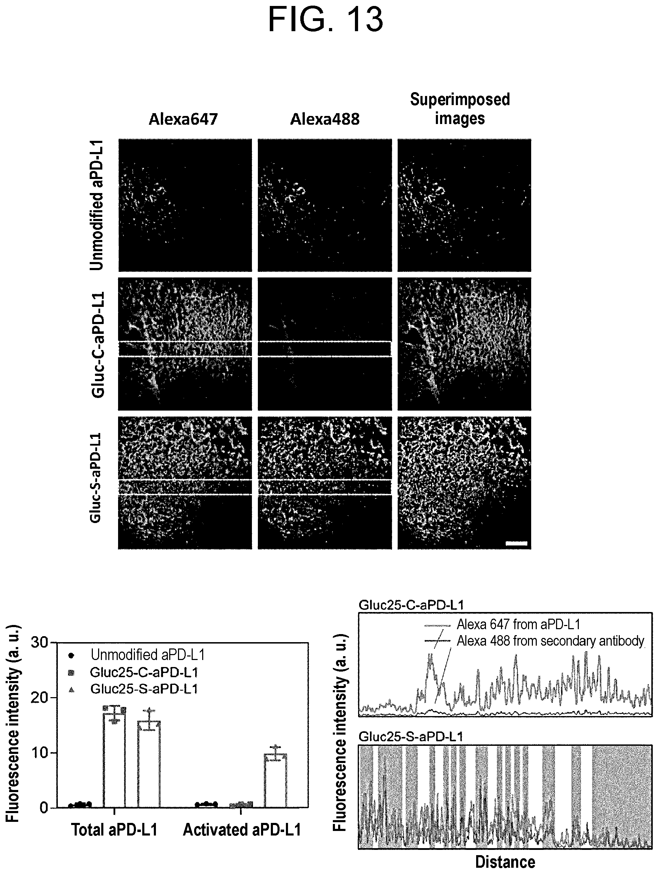

GSH and ascorbic acid (also called vitamin C) are considered to be the most potent central nervous system reducing factor against oxidative stress in the brain, and is present approximately at about millimolar level^{36,37}, and indispensable for maintenance of normal functions and subsistence of nerve cells. For directly observing reduction-induced cleavage of the PEG chain, Gluc-S-aPD-Ll and aPD-Ll released in response to reduction were distinguished using an Alexa 488-labeled anti-human secondary antibody. The Alexa 488-labeled anti-human secondary antibody binds to released aPD-Ll, but does not bind to Gluc-S-aPD-Ll because of the shell structure of the PEG chain (Figure 12). First, aPD-Ll was labeled with an Alexa 647 dye. Next, GlucO-S-aPD-Ll, Gluc25-S-aPD-L1 and Gluc25-C-aPD-L1 were each prepared. Next, the various preparations were intravenously injected into mice with an orthotopic brain tumor. After 24 hours, the mice were sacrificed, and tumor tissues were extracted. From the extracted tissues, tissue sections were prepared, and incubated with an anti-human secondary antibody. As shown in Figure 13, unmodified aPD-Ll having no active transportation ability to pass over BBB was minimally accumulated in the tumor site, whereas Gluc25-S-aPD-L1 and Gluc25-C-aPD-L1 were abundantly distributed in the brain due to introduction of glucose. Further, it was revealed that in Gluc25-S-aPD-Ll, the PEG chain was efficiently cleaved in response to a reducing environment to release aPD-Ll (lower left panel of Figure 13). Gluc-S-aPD-Ll labeled with green fluorescence exhibited a colocalization of 72% with total Gluc-S-aPD-Ll regardless of whether the PEG chain was cleaved or not. This reflects the fact that 72% of the capability of Gluc-S-aPD-Ll to bind to PD-L1 was recovered when a reducing signal was sensed. On the other hand, for the mice treated with Gluc25-C-aPD-L1, fluorescence of Alexa 488 was observed only at an ignorable level (lower right panel of Figure 13) because it was difficult to cleave the PEG chain. Therefore, the reducing environment-dependent PD-L1 release behavior of Gluc-S-aPD-Ll via cleavage of the PEG chain opens the way for the alleviation of side effects in areas other than brain tissues by an inactivated antibody, and ICB therapy based on reactivation in the brain.

### Induction of immune response by Gluc-S-aPD-Ll

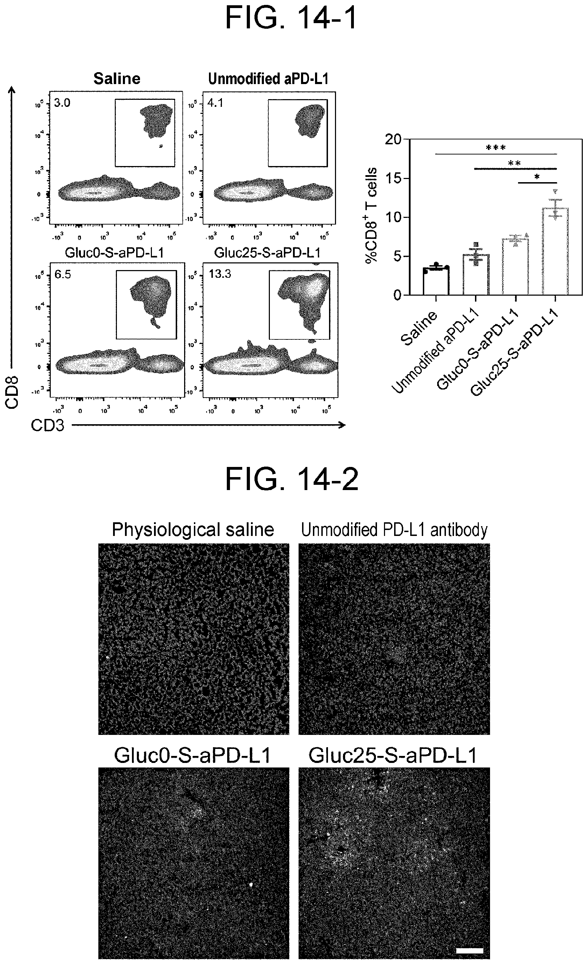

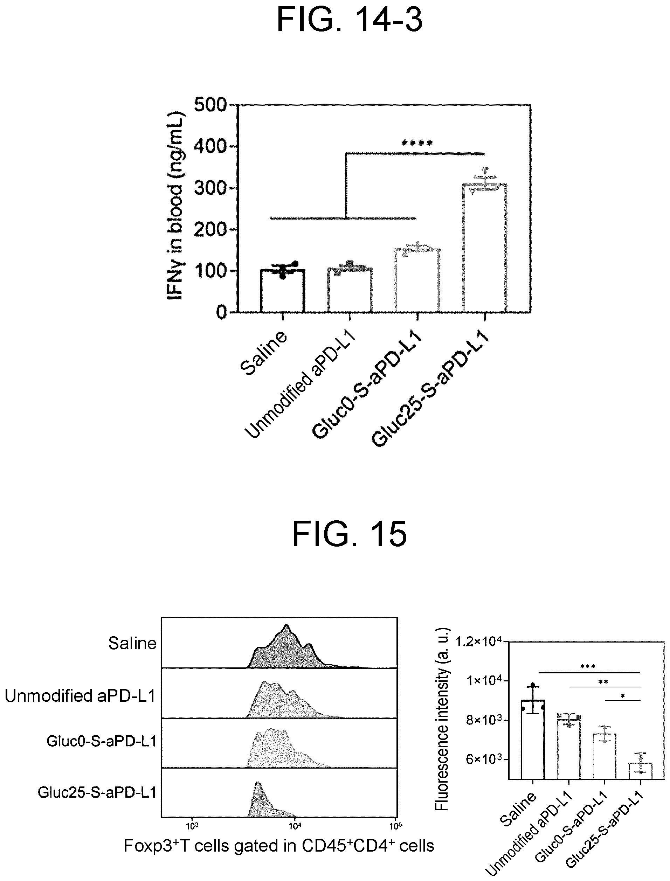

Three days after the injection, T-cells in the tumor were collected, and analyzed by flow cytometry for examining an immune response behind the therapeutic effect of Gluc25-S-aPD-L1. In the Gluc25-S-aPD-L1 administration group, the number of cytotoxic T-cells (CD8 + T-cells) considered to be prevailing T-cell populations for attacking tumor cells increased 2 to 3 times as compared to the physiological saline administration group, the aPD-Ll administration group and the GlucO-S-aPD-Ll administration group (Figure 14-1). This indicates that the strategy of targeting a tumor and reactivating an antibody in the brain in the present invention is effective for induction of a robust antitumor immune response. An increase in the number of infiltrating CD8 + T cells was also demonstrated by immunostaining of tumor sections from Gluc25-S-aPD-L1-treated mice (Figure 14-2). In addition, the release of IFN-γ typically promoting the T-cell response was tested by stimulating the antigen processing and presentation mechanism, and the results showed that the value in mice receiving Gluc25-S-aPD-L1 was 2 to 3 times higher than the value in other control groups (Figure 14-3). Further, the number of immunosuppressive Foxp3 + T-cell (regulatory T-cell, Treg) populations evidently decreased in Gluc25-S-aPD-L1 administration group (Figure 15) as compared to control groups. This suggests that the immunotherapeutic effect can be enhanced by relaxing immunosuppressive microenvironments. From this, it is considered that Gluc25-S-aPD-L1 can enhance an immune response by acting as a reactivatable immunotherapeutic agent, promoting infiltration of CD8 + T-cells and reducing Treg at the tumor site, and can be expected to have an antitumor effect.

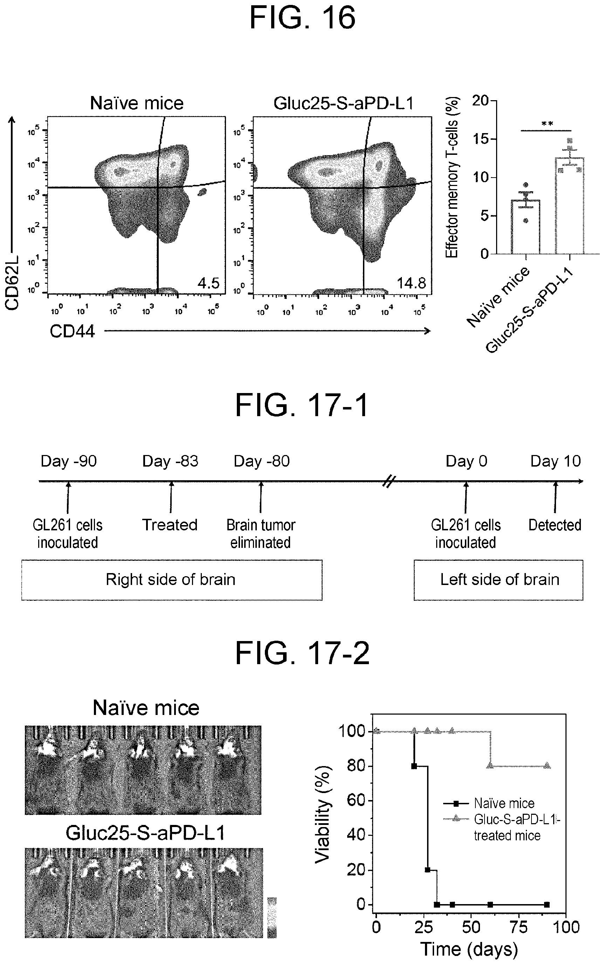

From the mice receiving Gluc25-S-aPD-L1, T-cells of the spleen were collected, and analyzed by flow cytometry for examining the priming of memory T-cells that promote effective prophylaxis of tumor recurrence. In the Gluc25-S-aPD-L1 administration group, the number of CD44hiCD62Llow effector memory T-cell subsets increased about 2.0 times as compared to native mice (Figure 16). The function of the effector memory T-cells was further confirmed by tumor rechallenge experiments. Mice having Gluc25-S-aPD-L1 administered to the right side of the brain to eliminate the tumor were rechallenged with GL261 tumor cells again to the left side of the brain (Figure 17-1). It was confirmed that as shown in Figure 17-2, tumor growth was suppressed while a non-tumor state was maintained in the Gluc25-S-aPD-L1 administration group, whereas the tumor rapidly grew within a survival duration of 25 days in native mice. These findings suggest that Gluc25-S-aPD-L1 can effectively prevent tumor recurrence for long-term survival.

### Suppression of onset of irAE by Gluc-S-aPD-Ll

It is reasonable to consider that in conventional techniques, PD-L1 is expressed on vascular endothelial walls, and therefore the first off-target process of aPD-L1 occurs in the blood circulation, resulting in induction of systemic inflammation²⁶. Thus, aPD-Ll released from Gluc25-S-aPD-L1 within 24 hours was quantitatively measured in the blood circulation. The results showed that a large amount of unmodified aPD-L1 exhibited normal binding affinity for PD-L1, but in the Gluc25-S-aPD-L1 group, off-target binding in a physiological environment was further avoided. This suggests that since the reduction level in the blood was limited, the amount of aPD-L1 released from Gluc25-S-aPD-L1 was small, and the strategy of PEGylation of aPD-L1 effectively blocked the binding affinity for the vascular endothelial walls (right panel of Figure 8).

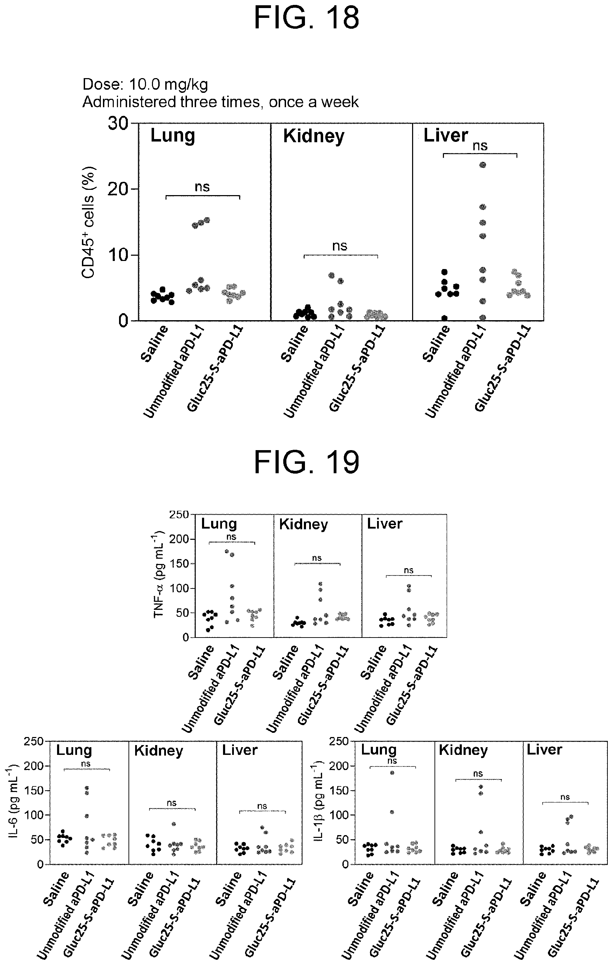

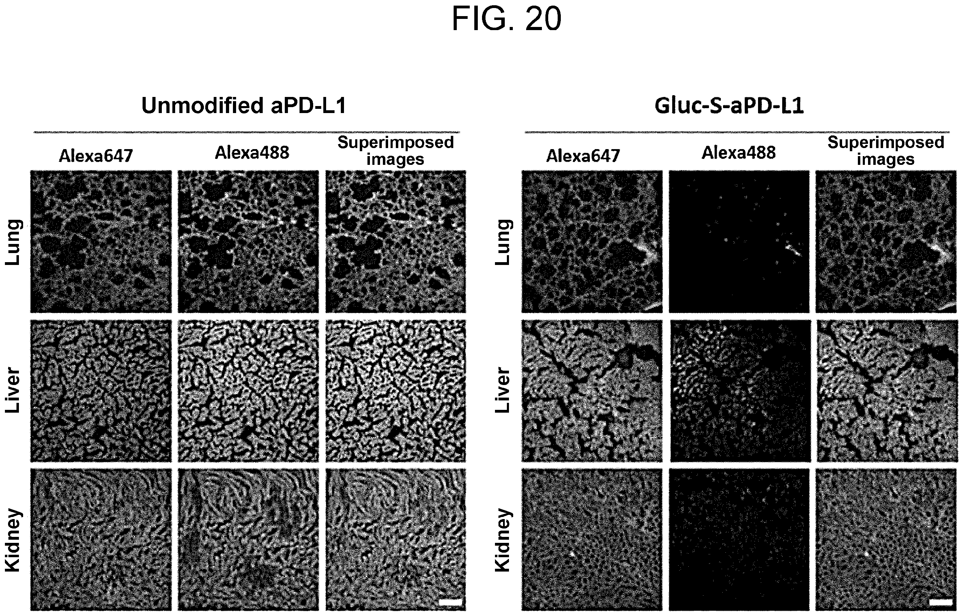

Further, for demonstrating the reduction of the off-target effect, non-target tissues (lung, liver, kidney and the like) were selected, and the possibility of suppressing immune-related adverse events (irAEs) was evaluated Gluc25-S-aPD-L1. Adverse events caused by the off-target effect are generally thought to involve infiltration of lymphocytes and production of cytokines due to excessive activation of immune systems in the parenchyma of non-target tissues¹¹⁻¹³. Upon injection of unmodified aPD-L1 and Gluc25-S-aPD-L1 after five days, the lung and the kidney were extracted for measuring the release of infiltrating lymphocytes (CD45 + cells) and inflammation promoting cytokines (for example, TNF-α, IL-6 and IL-1β). As shown in Figure 18, a significant improvement in infiltration of CD45 + cells was evidently detected in some of mice receiving unmodified aPD-L1, but this may be due to off-target binding affinity in normal tissues for inducing autoimmune activation. In contrast, in the mice of the Gluc25-S-aPD-L1 administration group, relatively low lymphocyte infiltration substantially equivalent to that in the physiological saline administration group occurred. This suggests that the inactivation function of Gluc25-S-aPD-L1 contributes to the reduced risk of excessive infiltration of lymphocytes in non-target tissues. Further, the tissues were homogenized, and the kinetics of inflammatory cytokines released in the non-target tissues was examined³⁸. The results showed that there was an abnormal increase of 2 to 4 times in TNF-**α**, IL-6 and IL-1**β** levels at 25% in the mice receiving unmodified aPD-L1 as compared to the physiological saline administration group and the Gluc25-S-aPD-L1 administration group (Figure 19). Further, the tissue sections were stained to examine whether aPD-L1 was released from Gluc25-S-aPD-L1 in non-target tissues (Figure 20). The unmodified aPD-L1 labeled with Alexa 647 in the control group is distributed in non-target tissues while colocalizing with green fluorescence, the binding affinity for PD-L1 causes off-target binding to PD-L1 expressed in a normal organ, and this is suggested to definitely lead to autoimmune activation (lower panel of Figure 20). However, in non-target tissues, Gluc25-S-aPD-L1 failed to release aPD-L1 due to the lack of a reducing environment, so that it was not possible to target PD-L1 in the non-target tissues. This may be the reason why Gluc25-S-aPD-L1 did not induce immune activation in the non-target tissues. From the above, it is suggested that adverse events in non-target tissues can be avoided by performing ICB therapy using the antibody of the present invention which is reactivatable in a brain tumor-specific manner, and it is possible to improve clinical performance while suppressing side effects.

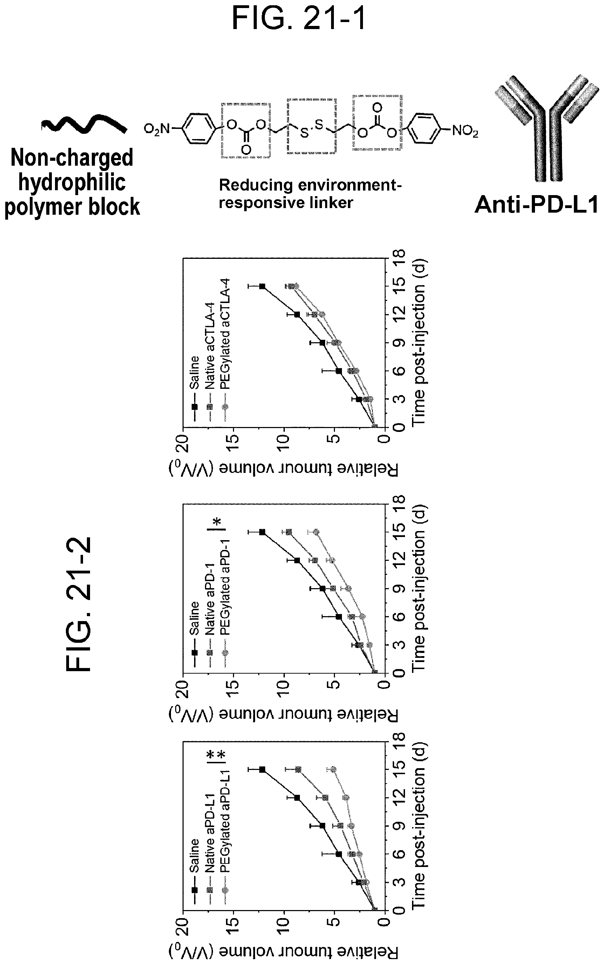

In Example described above, a GLUT1 ligand was linked to PEG as a brain targeting molecule for enhancing the efficiency of delivery of an antibody to the brain. In the following, a PEG-modified antibody having no targeting molecule was prepared for delivery of an antibody to areas other than the brain and cancer therapy experiments. As shown in Figure 21-1, the antibody used in the following is a modified antibody in which a non-charged hydrophilic polymer block, a reducing environment-responsive linker and the antibody are linked in this order. As the non-charged hydrophilic polymer block, PEG was used as described above. Due to the PEGylation, the antibody lost its antigen binding activity.

To mouse models of subcutaneous transplantation of malignant melanoma (B16-F10), physiological saline, an unmodified anti-PD-L1 antibody, and a PEG-modified anti-PD-L1 antibody having no targeting molecule were administered through the tail vein (n=5 for each experimental group). The PEG-modified anti-PD-L1 antibody was prepared in the same manner as in preparation of the antibody in Example described above. The dosage amount was adjusted to 1.5 mg/kg of body weight in terms of the antibody. The administration was performed three times every other day. The tumor volume (V) was measured on the first day and every fourth days, and the ratio of the tumor volume on the relevant day (V) to the tumor volume on the first day (VO) (V/VO) was determined.

The results were as shown in Figure 21-2. In the unmodified anti-PD-L1 antibody administration group (Native aPD-L1), a significant suppressing action on an increase in tumor volume was exhibited as compared to the physiological saline administration group (Saline) as shown in Figure 21-2. On the other hand, in the group receiving the reducing environment-responsive PEG-modified antibody of the present invention (PEGylated aPD-L1), a stronger suppressing action was exhibited on an increase in tumor volume as compared to the unmodified anti-PD-L1 antibody administration group (left panel of Figure 21-2). Next, similar experiments were conducted under the same conditions except that an anti-PD-1 antibody was used instead of the anti-PD-L1 antibody. The results were as shown in the center panel of Figure 21-2. The effect was also significant when the anti-PD-1 antibody was used. In the group receiving the reducing environment-responsive PEG-modified antibody of the present invention (PEGylated aPD-1), a stronger suppressing action was exhibited on an increase in tumor volume as compared to the unmodified anti-PD-1 antibody administration group (center panel of Figure 21-2). This tendency was also observed when as the antibody, an anti-CTLA-4 antibody was used.

The inside of a tumor tissue is generally a reducing environment. The tumor generally provides a low-oxygen environment due to deficiency in supply of nutrients and oxygen for cell growth. Even in a low-oxygen environment, the inside of the tumor tissue is a reducing environment because of an abundance of reducing enzymes present in cancer cells. In Example, the PEG-modified antibody was designed to return to a native antibody with PEG cleaved under a reducing environment-responsive manner. It was revealed that the antibody recovered lost antigen binding activity by actually responding to a reducing environment in the tumor *in vivo,* and exhibited the same effect as that of the unmodified antibody. The ability to bind to areas other than the tumor in a living organism may be eliminated by PEG modification, and therefore it is reasonably considered that side effects by the antibody outside the tumor tissue is reduced. On the other hand, however, the PEG-modified antibody of the present invention was equivalent or superior in antitumor effect to the unmodified antibody. This suggests that due to the modification, the amount of the antibody delivered to the tumor increased, that is, the EPR effect was enhanced. Thus, in the PEG-modified antibody of the present invention, its binding activity can be eliminated by the modification to reduce its side effects outside the tumor tissue, and/or the amount of delivery to the tumor tissue can be increased. This enables achievement of reduced side effects and/or an improved amount of delivery to the tumor tissue as compared to conventional unmodified antibodies.

### Application of the modification of the present invention to antibody-drug conjugate (ADC)

The modification according to the present invention can also be applied to an antibody-drug conjugate. As ADC, KADCYLA (Rosche, Ltd.) was used. KADCYLA is ADC represented by the following formula (I) and obtained by linking trastuzumab and emtansine via a linker (hereinafter, also referred to as "T-DM1"), and is used as an anticancer agent.

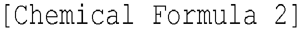

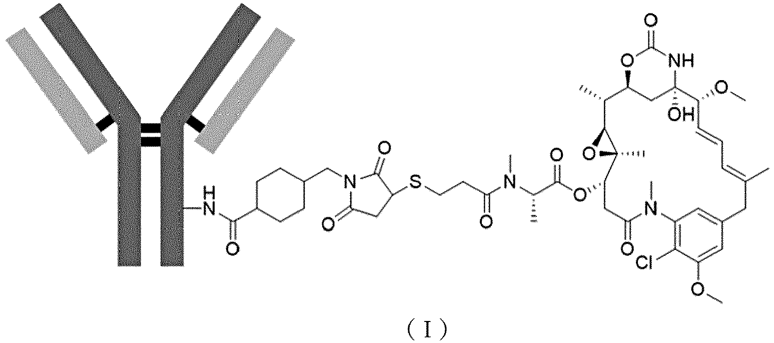

PEG modification was performed on the ADC (unmodified ADC) to obtain modified ADC. Specifically, the PEG modification was performed using PEG linked to a cleavable linker having the structure of R-PEG-(CH₂)₂-SS-(CH₂)₂-nitrophenyl carbonate (NPC) represented by the following formula (II). In the Example, R was a methoxy group. R can also be glucose, a reactive group (for example, an azide group) or the like. A targeting molecule for targeting a tissue in the body can be introduced into the reactive group.

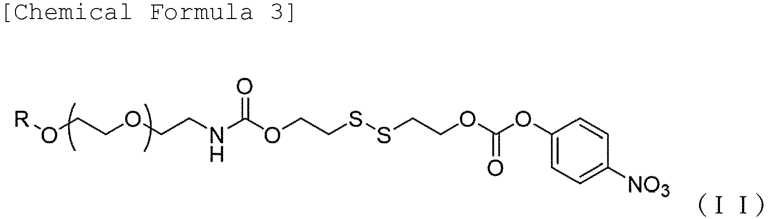

The present linker is linked to some of amino groups present in a large amount in the antibody, and under a reducing environment, cleavage occurs at the linker, so that ADC (unmodified ADC) is released from modified ADC. In the present test, studies were conducted on PEG modification of ADC and release of ADC (unmodified ADC) under a reducing environment.

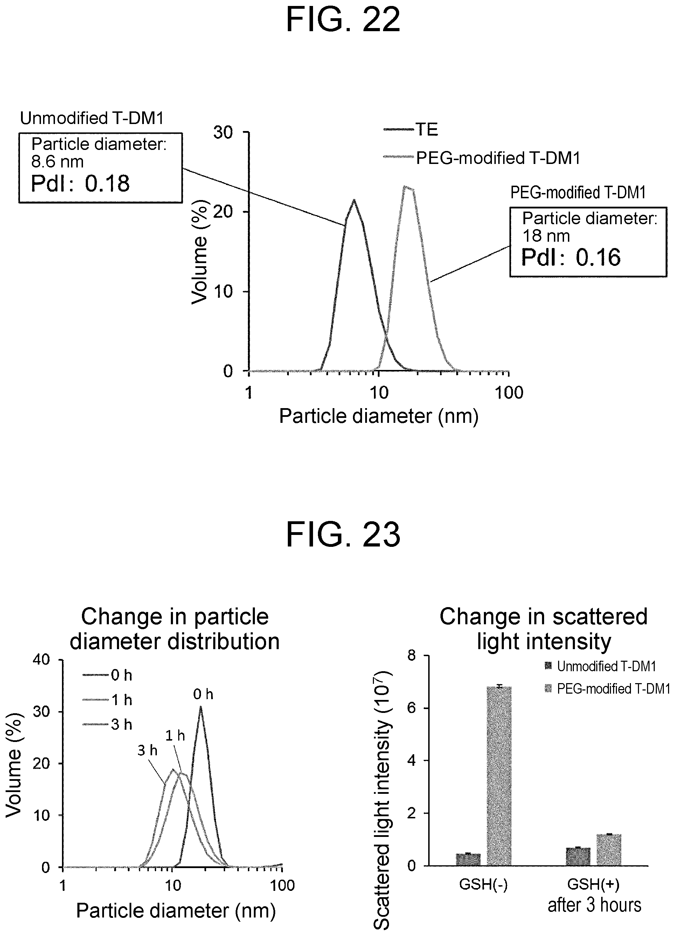

KADCYLA at 4.10 mg/ml (T-DM1 at 1.0 mg/ml) and a solution of PEG-(CH₂)₂-SS-(CH₂)₂-NPC at 10 mg/ml in a 20 mM phosphate buffer solution (pH 7.4) were mixed at 1 : 1 (volume ratio), and reacted overnight at room temperature. The reaction solution was purified five times by ultrafiltration (Vivaspin 6, MWCO: 30 kDa, 4,000 g, 4°C), and treated with a 0.1 µm PVDF filter to obtain PEG-modified T-DM1. The particle size distribution and the scattered light intensity were measured with Zetasizer Nano ZS (532 nm, 173 °). As a control, T-DM1 which was not modified with PEG (unmodified T-DM1) was measured. The results were as shown in Figure 22. The T-DM1 modified with PEG (PEG-modified T-DM1) evidently had an increased average hydrodynamic diameter (volume average) as compared to the unmodified T-DM1 as shown in Figure 22. This suggests that T-DM1 was modified with PEG.

It is known that the concentration of glutathione (GSH) is high in a cancer tissue. In the present test, PEG-modified T-DM1 was incubated in the presence of 1 mM GSH for simulating a reducing environment in a cancer tissue (n = 3 for each test). After 0, 1 or 3 hours, the particle size distribution and the scattered light intensity were measured as described above. As a control, unmodified T-DM1 was measured. The results were as shown in Figure 23. As shown in the left panel of Figure 23, the hydrodynamic diameter (volume average) of the PEG-modified T-DM1 decreased as the time of incubation in the presence of GSH increased. As shown in the right panel of Figure 23, the scattered light intensity of the PEG-modified T-DM1 decreased to substantially the same level of the scattered light intensity of the T-DM1 after incubation in the presence of GSH for 3 hours. Thus, the PEG-modified T-DM1 is cleaved under a reducing environment in the cancer tissue. It is suggested that as a result, the PEG-modified T-DM1 releases intact unmodified T-DM1.

### Discussions

As described above, herein, reactivatable ICB antibody delivery systems based on a target molecule non-modification type PEGylation process and a glucose modification type PEGylation process were developed. An appropriately configured PEG-modified antibody having a glucose molecule recognized GLUT1, so that preferential and efficient passage of BBB was successfully promoted. Even in the case of a target molecule non-modification type PEG-modified antibody, the antibody was equivalent or superior in antitumor effect to an unmodified antibody. The most attractive advantage of such a delivery system is that the antibody function can be switched on and off in the target tissue or the non-target tissue, and the PEG-modified antibody whose binding affinity is blocked detects a reducing environment in the tumor and the brain, and thus induces detachment of the PEG chain to recover the original ability to bind to an antigen of interest, whereby a strongly activated antitumor immune response can be derived in a brain tissue-specific manner. In this way, an antitumor effect can be exhibited on a tumor outside a brain tissue and primary glioblastoma, and memory immunity can be induced to prevent long-term tumor recurrence. In contrast, a PEG-modified antibody maintaining a perfect structure in the non-target tissue (lung, liver, kidney or the like) is designed not to induce generation of immune-related adverse events as the antigen specificity of the antibody is blocked. Up to now, during clinical ICB therapy using an unmodified immune checkpoint inhibition antibody, drug-related adverse events have been observed in 39% of patients, and events of grade 3 or 4 have been pointed out in 9% of patients³⁹. Thus, the strategy of activatable ICB therapy herein reduces the incidence of irAE in the future, and promotes clinical application which may produce advantageous results against glioblastoma.

As demonstrated by some FDA-approved drugs such as Doxil²³, the PEGylation process comprising linkage of a PEG chain to a polypeptide, another candidate molecules or nanocarriers is a long-standing strategy for overcoming shortcomings of medicaments such as rapid elimination, pseudo-allergy reactions and insufficient target accumulation. In view of such promising application of activatable ICB therapy, the strategy in the present invention using a PEGylated antibody can be utilized as a common and versatile platform. For example, reaction properties such as acidic pH, oxidative stress and excessively expressed enzymes in TME can be activated in a site-specific manner by appropriate chemical design⁴⁰. Further, it is reasonable to select an appropriate ligand and antibody (including a biopolymer inhibitor and a cytokine) depending on a proposed target site. Therefore, the design of a PEGylated antibody has enormous potentialities for efficient protein delivery, and may further extend to design of a therapeutic protein for therapy of various diseases on the basis of physiological signals.

### Reference

17. Ye, Y., et al. Synergistic transcutaneous immunotherapy enhances antitumour immune responses through delivery of checkpoint inhibitors. ACS Nano 10, 8956-8963 (2016).
18. Chen, Q., et al. In situ sprayed bioresponsive immunotherapeutic gel for post-surgical cancer treatment. Nat. Nanotechnol. 14, 89-97 (2019).
19. Mi, Y., et al. A dual immunotherapy nanoparticle improves T-cell activation and cancer immunotherapy. Adv. Mater. 30, 1706098 (2018).
20. Bu, J., et al. An avidity-based PD-L1 antagonist using nanoparticle-antibody conjugates for enhanced immunotherapy. Nano Lett. 20, 4901-4909 (2020).
21. Hu, Q., et al. Conjugation of haematopoietic stem cells and platelets decorated with anti-PD-1 antibodies augments anti-leukaemia efficacy. Nat. Biomed. Eng. 2, 831-840 (2018).
22. Zhang, Y., et al. A tumour-targeted immune checkpoint blocker. Proc. Natl. Acad. Sci. 116, 15889-15894 (2019).
23. Harris, J.M. & Chess, R.B. Effect of pegylation on pharmaceuticals. Nat. Rev. Drug Discovery 2, 214-221 (2003).
24. Greenfield, N.J. Using circular dichroism spectra to estimate protein secondary structure. Nat. Protoc. 1, 2876-2890 (2006).
25. Louis-Jeune, C., Andrade-Navarro, M.A. & Perez-Iratxeta, C. Prediction of protein secondary structure from circular dichroism using theoretically derived spectra. Proteins 80, 374-381 (2012).
26. Wang, D., et al. Engineering nanoparticles to locally activate T cells in the tumour microenvironment. Sci. Immunol. 4, eaau6584 (2019).
27. Zhu, A., et al. Dually pH/reduction-responsive vesicles for ultrahigh-contrast fluorescence imaging and thermo-chemotherapy-synergized tumour ablation. ACS Nano 9, 7874-7885 (2015).
28. Wainwright, D.A., et al. Durable therapeutic efficacy utilizing combinatorial blockade against IDO, CTLA-4, and PD-L1 in mice with brain tumours. Clin. Cancer Res. 20, 5290-5301 (2014).
29. Thorens, B. & Mueckler, M. Glucose transporters in the 21st Century. Am. J. Physiol. 298, E141-E145 (2010).
30. Suzuki, K., et al. Glucose transporter 1-mediated vascular translocation of nanomedicines enhances accumulation and efficacy in solid tumours. J. Controlled Release 301, 28-41 (2019).
31. Anraku, Y., et al. Glycaemic control boosts glucosylated nanocarrier crossing the BBB into the brain. Nat. Commun. 8, 1001 (2017).
32. Xie, J., et al. Dual-sensitive nanomicelles enhancing systemic delivery of therapeutically active antibodies specifically into the brain. ACS Nano 14, 6729-6742 (2020).
33. Min, H.S., et al. Systemic brain delivery of antisense oligonucleotides across the blood-brain barrier with a glucose-coated polymeric nanocarrier. Angew. Chem. Int. Ed. 59, 8173-8180 (2020).
34. Cheng, F. & Eng, C. PTEN mutations trigger resistance to immunotherapy. Trends. Mol. Med. 25, 461-463 (2019).
35. Bao, S., et al. Glioma stem cells promote radioresistance by preferential activation of the DNA damage response. Nature 444, 756-760 (2006).
36. Sun, X., et al. Two-photon imaging of glutathione levels in intact brain indicates enhanced redox buffering in developing neurons and cells at the cerebrospinal fluid and blood-brain interface. J. Biol. Chem. 281, 17420-17431 (2006).
37. Harrison, F.E. & May, J.M. Vitamin C function in the brain: vital role of the ascorbate transporter SVCT2. Free Radical Biol. Med. 46, 719-730 (2009).
38. Ma, Q., et al. Calming cytokine storm in pneumonia by targeted delivery of TPCA-1 using platelet-derived extracellular vesicles. Matter 3, 287-301 (2020).
39. Brahmer, J.R., et al. Safety and activity of Anti-PD-L1 antibody in patients with advanced cancer. N. Engl. J. Med. 366, 2455-2465 (2012).
40. Blum, A.P., et al. Stimuli-responsive nanomaterials for biomedical applications. J. Am. Chem. Soc. 137, 2140-2154 (2015).

## Claims

1. A modified antibody that is modified with a non-charged hydrophilic polymer block, wherein the non-charged hydrophilic polymer block, an environment-responsive bond and the antibody are linked in this order, each of the linkages is optionally via a spacer, and the environment-responsive bond is cleaved under a reducing environment.

2. The antibody according to claim 1, wherein a binding affinity (KD) for an antigen of interest is 10% or less of that before modification.

3. The antibody according to claim 1, wherein a binding affinity (KD) for an antigen of interest is 5% or less of that before modification.

4. The antibody according to claim 1, which does not substantially or significantly bind to an antigen of interest under a serum environment.

5. The antibody according to any one of claims 1 to 4, wherein the environment-responsive bond is cleaved under a reducing environment in a brain parenchyma or under a reducing environment in a tumor tissue.

6. The antibody according to claim 5, wherein when the environment-responsive bond is cleaved, the binding affinity (KD) for an antigen of interest is recovered.

7. The antibody according to any one of claims 1 to 6, wherein the antibody has an amino group modified with - C(O)-O-L₁-S-S-L₂- (a non-charged hydrophilic polymer block),
wherein
L₁ is an optionally substituted lower alkylene, and
L₂ is a bond, or a linker stable in a living organism.

8. The antibody according to claim 7, wherein L₁ is ethylene.

9. The antibody according to any one of claims 1 to 8, wherein the antibody inhibits a PD-1-axis immune checkpoint.

10. The antibody according to claim 9, wherein the antibody binds to PD-L1.

11. The antibody according to any one of claims 1 to 8, wherein the antibody binds to a cancer antigen.

12. The antibody according to any one of claims 1 to 11, wherein the antibody exposes a targeting molecule.

13. The antibody according to claim 12, wherein the targeting molecule is linked to the non-charged hydrophilic polymer block.

14. The antibody according to claim 13, wherein the targeting molecule is a GLUT1 ligand.

15. The antibody according to claim 14, wherein the GLUT1 ligand is glucose.

16. The antibody according to any one of claims 1 to 15, wherein the antibody that binds to an immune checkpoint molecule to inhibit an immune checkpoint is an antibody that binds to an immune checkpoint molecule to neutralize interaction between the immune checkpoint molecules.

17. The antibody according to claim 16, wherein the immune checkpoint molecule is a counterpart of an immune checkpoint molecule expressed in immune cells.

18. The antibody according to claim 16, wherein the immune checkpoint molecule is an immune checkpoint molecule expressed in immune cells.

19. The antibody according to any one of claims 1 to 18, wherein the antibody is in a form of an antibody-drug conjugate (ADC).

20. A pharmaceutical composition comprising the antibody according to any one of claims 1 to 19.
